# EUROPEAN PATENT SPECIFICATION

(11) **EP 3 707 246 B1**
(45) Date of publication and mention of the grant of the patent: **07.06.2023**
(21) Application number: 18819234.8
(22) Date of filing: 07.11.2018
(51) Int. Cl.: C12N 5/0783, A61K 35/17, A61K 39/00, A61P 35/02

(54) **TREATMENT OF CNS LYMPHOMA AND SYSTEMIC LYMPHOMA WITH INTRACEREBROVENTRICULARLY ADMINISTERED CD19 CAR**
BEHANDLUNG VON ZNS-LYMPHOM UND SYSTEMISCHEM LYMPHOM MIT INTRACEREBROVENTRIKULÄR VERABREICHTEM CD19-CAR
TRAITEMENT DU LYMPHOME DU SNC ET LYMPHOME SYSTÉMIQUE AVEC CD19 CAR ADMINISTRÉ PAR VOIE INTRACÉRÉBROVENTRICULAIRE

(30) Priority: 07.11.2017 US 201762582891 P
(43) Date of publication of application: 16.09.2020
(73) Proprietor: City of Hope, Duarte, CA 91010 (US)
(72) Inventor: WANG, Xiuli, Temple City California 91780 (US); FORMAN, Stephen J., Duarte California 91010 (US)
(74) Representative: Fish & Richardson P.C.
(86) International application number: PCT/US2018/059668
(87) International publication number: WO 2019/094498

(56) References cited:
- LIREN Q ET AL: "Advances in the treatment of newly diagnosed primary central nervous system lymphomas", BLOOD RESEARCH, vol. 52, no. 3, 25 September 2017 (2017-09-25), pages 159-166, XP055565001, ISSN: 2287-979X, DOI: 10.5045/br.2017.52.3.159
- WANG X ET AL: "Phase 1 studies of central memory-derived CD19 CAR T-cell therapy following autologous HSCT in patients with B-cell NHL", BLOOD, vol. 127, no. 24, 16 June 2016 (2016-06-16), pages 2980-2990, XP055564969, ISSN: 0006-4971, DOI: 10.1182/blood-2015-12-686725 cited in the application
- BROWN C E ET AL: "Bioactivity and Safety of IL13Ralpha2-Redirected Chimeric Antigen Receptor CD8+ T Cells in Patients with Recurrent Glioblastoma", CLINICAL CANCER RESEARCH, vol. 21, no. 18, 15 September 2015 (2015-09-15), pages 4062-4072, XP055362794, ISSN: 1078-0432, DOI: 10.1158/1078-0432.CCR-15-0428
- BROWN C E ET AL: "Regression of Glioblastoma after Chimeric Antigen Receptor T-Cell Therapy", THE NEW ENGLAND JOURNAL OF MEDICINE, vol. 375, no. 26, 29 December 2016 (2016-12-29), pages 2561-2569, XP055564981, ISSN: 0028-4793, DOI: 10.1056/NEJMoa1610497
- PRICEMAN S J ET AL: "Regional Delivery of Chimeric Antigen Receptor-Engineered T Cells Effectively Targets HER2 + Breast Cancer Metastasis to the Brain", CLINICAL CANCER RESEARCH, vol. 24, no. 1, 23 October 2017 (2017-10-23), pages 95-105, XP055564978, ISSN: 1078-0432, DOI: 10.1158/1078-0432.CCR-17-2041
- GUST J ET AL: "Endothelial Activation and Blood-Brain Barrier Disruption in Neurotoxicity after Adoptive Immunotherapy with CD19 CAR-T Cells", CANCER DISCOVERY, vol. 7, no. 12, 12 October 2017 (2017-10-12), pages 1404-1419, XP055462614, ISSN: 2159-8274, DOI: 10.1158/2159-8290.CD-17-0698
- WENG J ET AL: "A novel generation 1928zT2 CAR T cells induce remission in extramedullary relapse of acute lymphoblastic leukemia", JOURNAL OF HEMATOLOGY & ONCOLOGY, vol. 11, 25, 20 February 2018 (2018-02-20) , XP055564973, DOI: 10.1186/s13045-018-0572-x
- WANG X ET AL: "New therapeutic approach for central nervous system lymphoma by CD19CAR T cells", JOURNAL FOR IMMUNOTHERAPY OF CANCER, vol. 5, no. Suppl. 2, P202, 7 November 2017 (2017-11-07), pages 108-109, XP055564996, 32nd Annual Meeting of the Society for Immunotherapy of Cancer; National Harbor, MD, USA, 8-12 November 2017 ISSN: 2051-1426, DOI: 10.1186/s40425-017-0289-3

## Description

### BACKGROUND

Tumor-specific T cell based immunotherapies, including those that employ engineered T cells and *ex vivo* expanded or selected T cells, have been investigated for anti-tumor treatment. In some cases, the T cells used in such therapies do not remain active *in vivo* for a long enough period. In some cases, the tumor-specificity of the T cells is relatively low. In some cases, the engineered T cells have insufficient access to the tumor. Therefore, there is a need in the art for tumor-specific cancer therapies with more effective anti-tumor function.

T cells that are genetically engineered with chimeric antigen receptors (CARs) targeting CD19 (CD19 CAR T cells) have broad application for adoptive therapy of B cell lineage malignancies and have shown tremendous potential in treatment of systemic lymphoma (Wang et al. 2016 Blood 127:2980). In CD19 CAR T cell trials, the CAR T cell are administered intravenously. CD19 CAR T cell trafficking in cerebrospinal fluid (CSF) has been reported but most if not all protocols exclude patients with active CNS involvement. Liren et al. describe in Blood Research, vol. 52, no. 3, 25 September 2017, on pages 159-166 advances in the treatment of newly diagnosed primary central nervous system lymphomas. Brown et al. describe in Clinical Cancer Research, vol. 21, no. 18, 15 September 2015, on pages 4062-4072 the bioactivity and safety of IL13Ralpha2-redirected chimeric antigen receptor CD8+ T cells in patients with recurrent glioblastoma.

Brown et al. describe in The New England Journal of Medicine, vol. 375, no. 26, 29 December 2016, on pages 2561-2569 a regression of glioblastoma after chimeric antigen receptor T cell therapy.

Gust et al. describe in Cancer Discovery, vol. 7, no. 12, 12 October 2017, on pages 1404-1419 an endothelial activation and blood-brain barrier disruption in neurotoxicity after adoptive immunotherapy with CD19 CAR-T cells.

### SUMMARY

The present invention is defined by independent claim 1. The dependent claims depict additional embodiments of the invention.

Described herein are compositions comprising CD19 CAR T cells for use in methods for treating B cell lymphoma or B cell leukemia of the central nervous system as well as peripheral B cell lymphoma or B cell leukemia by administering the compositions to the cerebrospinal fluid ("CSF") of a patient. Administration to the CNS can be accomplished, for example, by administration to the ventricular system or the central cavity of the spinal column. Administration to the CNS, as the term is used herein, is distinct from both intratumoral administration (injection or infusion into the tumor itself) and administration to a cavity created by resection of a tumor. In some cases, administration is to the ventricular system or the central cavity of the spinal column and not intratumoral and/or intracavity. However, the CNS administration methods described herein can be combined with intraturmoral and/or post-resection, intracavity administration. Surprisingly, the CAR T cells injected into the CSF are effective against peripheral lymphoma and, in some cases, are more effective against peripheral lymphoma than CAR T cells administered intravenously.

The CNS administration described herein permits, but does not require, infusion of relatively large volumes of the composition comprising T cells, for example 1 ml - 2 ml or more in a single infusion. Thus, several million T cells can be administered in a single infusion.

Described herein is a composition comprising an effective amount of T cells expressing a CD 19 targeted CAR for use in a method of treating a patient diagnosed with a CNS lymphoma or leukemia and/or peripheral lymphoma or leukemia, e.g., a B cell lymphoma or a B cell leukemia, comprising introducing into the cerebrospinal fluid (CSF) of a patient said composition comprising an effective amount of said T cells.

In various embodiments: the T cells are autologous or allogenic T cells; the T cells have been manipulated *ex vivo* by one or more of: expansion, fractionation or transfection with a recombinant nucleic acid molecule; the T cells comprise cells that have been transfected with a recombinant nucleic acid molecule encoding a polypeptide that binds to a CD19; the polypeptide is a chimeric antigen receptor; the composition is administered intraventricularly; the composition is administered to the central canal of the spinal cord; the administration is to the left ventrical or the right ventrical; the composition comprises at least 1 × 10⁶ cells; the composition comprising T cells is administered at least two times; the administrations differ in the total number of T cells administered; the administrations escalate in dose; the administrations de-escalate in dose; the T cells comprise CAR T cells targeting CD19; the T cells comprise autologous tumor infiltrating lymphocytes targeting CD19; the T cells comprise TCR-engineered T cells targeting CD19; the malignancy is a diffuse, infiltrating tumor; the malignancy is a primary brain tumor; one or more tumor foci decrease in size by at least 25%; the malignancy arose from a primary cancer; the method is performed after tumor resection; the method further comprises intratumoral administration of a composition comprising T cells; the malignancy is secondary brain tumor; the method further comprises intratumoral administration of a composition comprising therapeutic T cells expressing a chimeric antigen receptor that binds to CD19; T cells comprise both CD4+ cells and CD8+ cells; the T cells have undergone *ex vivo* expansion; the T cells comprise at least 10%, 20%, 30% or 40% central memory T cells (T_{CM} cells); the T cells comprise at least 10%, 20%, 30% or 40% naive T cells/memory T cells (T_{N/MEM} cells); at least 40%, 50%, 60%, 70% or more of the cells infused are CD4+; at least at least 40%, 50%, 60%, 70% or more of the cells infused express a cell surface receptor that targets CD19; and the dose of cells is based on the number of infused cells that express a cell surface receptor that targets CD19.

In some embodiments the T cells comprise CAR T cells that target CD19 and the cells comprise a nucleic acid molecule encoding a chimeric antigen receptor comprising: scFV targeted to CD 19 or a variant thereof having 1-10 (e.g., no more than 5 or no more than 2) amino acid modifications; a transmembrane domain selected from: a CD4 transmembrane domain or variant thereof having 1-10 (e.g., no more than 5 or no more than 2) amino acid modifications, a CD8 transmembrane domain or variant thereof having 1-10 (e.g., no more than 5 or no more than 2) amino acid modifications, a CD28 transmembrane domain or a variant thereof having 1-10 (e.g., no more than 5 or no more than 2) amino acid modifications, and a CD3ζ transmembrane domain or a variant thereof having 1-10 (e.g., no more than 5 or no more than 2) amino acid modifications; at least one costimulatory domain; and CD3 ζ signaling domain of a variant thereof having 1-10 (e.g., no more than 5 or no more than 2) amino acid modifications. In some embodiments: the costimulatory domain is selected from the group consisting of: a CD28 costimulatory domain or a variant thereof having 1-10 (e.g., no more than 5 or no more than 2) amino acid modifications, a 4IBB costimulatory domain or a variant thereof having 1-10 (e.g., no more than 5 or no more than 2) amino acid modifications and an OX40 costimulatory domain or a variant thereof having 1-10 (e.g., no more than 5 or no more than 2) amino acid modifications; the scFv targeted to CD19 comprises the amino acid sequence of SEQ ID NO:3 or SEQ ID NO:3 with 1 to 5 amino acid modifications; the chimeric antigen receptor comprises two different costimulatory domains selected from the group consisting of: a CD28 costimulatory domain or a variant thereof having 1-10 (e.g., no more than 5 or no more than 2) amino acid modifications, a 4IBB costimulatory domain or a variant thereof having 1-10 (e.g., no more than 5 or no more than 2) amino acid modifications and an OX40 costimulatory domain or a variant thereof having 1-10 (e.g., no more than 5 or no more than 2) amino acid modifications; the chimeric antigen receptor comprises two different costimulatory domains selected from the group consisting of: a CD28 costimulatory domain or a variant thereof having 1-2 amino acid modifications, a 4IBB costimulatory domain or a variant thereof having 1-2 amino acid modifications and an OX40 costimulatory domain or a variant thereof having 1-2 amino acid modifications; the chimeric antigen receptor comprises: CD19 scFv or a variant thereof having 1-2 amino acid modifications; a transmembrane domain selected from: a CD4 transmembrane domain or variant thereof having 1-2 amino acid modifications, a CD8 transmembrane domain or variant thereof having 1-2 amino acid modifications, a CD28 transmembrane domain or a variant thereof having 1-2 amino acid modifications, and a CD3ζ transmembrane domain or a variant thereof having 1-2 amino acid modifications; a costimulatory domain; and CD3 ζ signaling domain of a variant thereof having 1-2 amino acid modifications; the CAR comprises a spacer region located between the CD19 scFv or variant thereof and the transmembrane domain; the spacer region comprises an amino acid sequence selected from the group consisting of SEQ ID NO: 11-21; the chimeric antigen receptor comprises an amino acid sequence selected from the group consisting of any of SEQ ID NOs: 10, 1, 2, 4 and 5 or any of SEQ ID NOs: 10, 1, 2, 4 and 5 having no more than 5 (e.g., 4, 3, 2, or 1) single amino acid substitutions.

TIL are tumor infiltrating lymphocytes that can be isolated from a patient or a donor, expand ex vivo and re-infused into the patient in need thereof.

CAR T cells express chimeric T cell receptors that comprise an extracellular domain, a transmembrane region and an intracellular signaling domain. The extracellular domain includes a portion that binds the targeted cell and, optionally, a spacer, comprising, for example a portion human Fc domain. The transmembrane portion includes suitable transmembrane domain, for example, a CD4 transmembrane domain, a CD8 transmembrane domain, a CD28 transmembrane domain, a CD3 transmembrane domain or a 4IBB transmembrane domain. The intracellular signaling domain includes the signaling domain from the zeta chain of the human CD3 complex (CD3ζ) and one or more costimulatory domains, e.g., a 4-1BB costimulatory domain. The target cell binding portion of extracellular domain (for example a scFv targeted to CD19) enables the CAR, when expressed on the surface of a T cell, to direct T cell activity to those cells expressing CD 19.

A variety of different T cells, for example, patient-specific, autologous T cells, can be engineered to express a TCR or a CAR targeted to CD19. Various T cell subsets can be used. In addition, CAR can be expressed in other immune cells such as NK cells. Where a patient is treated with an immune cell expressing a CAR or TCR the cell can be an autologous or allogenic T cell. In some cases, the cells used are CD4+ and CD8+ central memory T cells (T_{CM}), which are CD45RO+CD62L+. This cell population is enriched for CD62L expressing cells and substantially depleted for CD45RA expressing cells, CD14 expressing cells and CD25 expressing cells. The T_{CM} cells can include CD4+ cells and CD8+ cells.

In some cases, the cells used comprise a mixture of naive T cells (T_{N}) and memory T cells (T_{MEM}). Memory T cells include central memory T cells (T_{CM}) and stem central memory cells (T_{SCM}). Thus, the T cell population that is a mixture of naive T cells, central memory T cells and stem central memory T cells can be referred to as "T_{N/MEM}" or "T_{CM/SCM/N}" and these two notations are used interchangeably herein. This cell population is enriched for CD62L expressing cells and substantially depleted for CD14 expressing cells and CD25 expressing cells. Thus, a population of cells comprising (e.g., more that 50%, 60%, 70%, 80%, 90% or 95%) CD62L+ T cells can be administered.

The T cells used in the methods described herein can contain a nucleic acid molecule encoding a chimeric antigen receptor (CAR), wherein the chimeric antigen receptor comprises: scFv targeted to CD19 or a variant thereof having 1-10 (e.g., 1 or 2) amino acid modifications; a transmembrane domain selected from: a CD4 transmembrane domain or variant thereof having 1-10 (e.g., 1 or 2) amino acid modifications, a CD8 transmembrane domain or variant thereof having 1-10 (e.g., 1 or 2) amino acid modifications, a CD28 transmembrane domain or a variant thereof having 1-10 (e.g., 1 or 2) amino acid modifications, and a CD3ζ transmembrane domain or a variant thereof having 1-10 (e.g., 1 or 2) amino acid modifications; a costimulatory domain; and CD3 ζ signaling domain of a variant thereof having 1-10 (e.g., 1 or 2) amino acid modifications.

The inclusion of a costimulatory domain, such as the 4-1BB (CD137) or CD28 costimulatory domain in series with CD3ζ in the intracellular region enables the T cell to receive costimulatory signals. Thus, in various embodiments, the costimulatory domain is selected from the group consisting of: a CD28 costimulatory domain or a variant thereof having 1-10 (e.g., 1 or 2) amino acid modifications, a 4-IBB costimulatory domain or a variant thereof having 1-10 (e.g., 1 or 2) amino acid modifications and an OX40 costimulatory domain or a variant thereof having 1-10 (e.g., 1 or 2) amino acid modifications. In certain embodiments, a 4IBB costimulatory domain or a variant thereof having 1-10 (e.g., 1 or 2) amino acid modifications is present.

In additional embodiments of the methods, the CAR expressed by the T cells comprises: a variant a scFv targeted to CD19 having 1-10 (e.g., 1 or 2) amino acid modifications compared to SEQ ID NO: 3; two different costimulatory domains selected from the group consisting of: a CD28 costimulatory domain or a variant thereof having 1-10 (e.g., 1 or 2) amino acid modifications, a 4IBB costimulatory domain or a variant thereof having 1-10 (e.g., 1 or 2) amino acid modifications and an OX40 costimulatory domain or a variant thereof having 1-10 (e.g., 1 or 2) amino acid modifications; two different costimulatory domains selected from the group consisting of: a CD28 costimulatory domain or a variant thereof having 1-2 amino acid modifications, a 4IBB costimulatory domain or a variant thereof having 1-2 amino acid modifications and an OX40 costimulatory domain or a variant thereof having 1-2 amino acid modifications; human IL-13 or a variant thereof having 1-2 amino acid modifications; a transmembrane domain selected from: a CD4 transmembrane domain or variant thereof having 1-2 amino acid modifications, a CD8 transmembrane domain or variant thereof having 1-2 amino acid modifications, a CD28 transmembrane domain or a variant thereof having 1-2 amino acid modifications, and a CD3ζ transmembrane domain or a variant thereof having 1-2 amino acid modifications; a costimulatory domain; and CD3ζ signaling domain of a variant thereof having 1-2 amino acid modifications; a spacer region located between the IL-13 or variant thereof and the transmembrane domain (e.g., the spacer region comprises an amino acid sequence selected from the group consisting of SEQ ID NO: 11-22); the spacer comprises an IgG hinge region; the spacer region comprises 10-150 amino acids; the 4-1BB signaling domain comprises the amino acid sequence of SEQ ID NO:33; the CD3ζ signaling domain comprises the amino acid sequence of SEQ ID NO:30; and a linker of 3 to 15 amino acids that is located between the costimulatory domain and the CD3 ζ signaling domain or variant thereof. In certain embodiments where there are two costimulatory domains, one is a 4-IBB costimulatory domain and the other a costimulatory domain selected from: CD28 and CD28gg

In some embodiments of the methods described herein the T cells harbor a nucleic acid molecule that expresses a polypeptide comprising an amino acid sequence selected from SEQ ID NOs: 10, 1, 2, 4 and 5; the chimeric antigen receptor comprises a CD19 scFv region comprising the amino acid of SEQ ID NO:3 and a CD3 ζ signaling domain comprising the amino acid sequence of SEQ ID NO:30; and the chimeric antigen receptor comprises the amino acid sequence of any of SEQ ID NOs: 10, 1, 2, 4 and 5.

Also disclosed are methods comprising intraventricular administration of a population of human T cells transduced by a vector comprising an expression cassette encoding a chimeric antigen receptor, wherein chimeric antigen receptor comprises: human IL-13 or a variant thereof having 1-10 amino acid modifications; a transmembrane domain selected from: a CD4 transmembrane domain or variant thereof having 1-10 amino acid modifications, a CD8 transmembrane domain or variant thereof having 1-10 amino acid modifications, a CD28 transmembrane domain or a variant thereof having 1-10 amino acid modifications, and a CD3ζ transmembrane domain or a variant thereof having 1-10 amino acid modifications; a costimulatory domain; and CD3 ζ signaling domain of a variant thereof having 1-10 amino acid modifications. In various embodiments: the population of human T cells comprise a vector expressing a chimeric antigen receptor comprising an amino acid sequence selected from SEQ ID NOs: 10, 1, 2, 4 and 5; the population of human T cells are comprises of central memory T cells (T_{CM} cells) (e.g., at least 20%, 30%, 40%, 50% 60%, 70%, 80% of the cells are T_{CM} cells; at least 10%, 15%, 20%, 25%, 30% or 35% of the T cells or the T_{CM} cells are CD4+ and at least 10%, 15%, 20%, 25%, 30% or 35% of the T cells or the T_{CM} cells are CD8+ cells).

The population of human T cells comprises T_{N/MEM} (T_{CM} cells) (e.g., at least 20%, 30%, 40%, 50% 60%, 70%, 80% of the cells are T_{N/MEM} cells; at least 10%, 15%, 20%, 25%, 30% or 35% of the T cells or the T_{N/MEM} cells are CD4+ and at least 10%, 15%, 20%, 25%, 30% or 35% of the T cells or the T_{N/MEM} cells are CD8+ cells).

Also described is a method of treating cancer in a patient comprising CNS administration of a population of autologous or allogeneic human T cells (e.g., autologous or allogeneic T cells comprising T_{CM} cells, e.g., at least 20%, 30%, 40%, 50% 60%, 70%, 80% of the cells are T_{CM} or T_{N/MEM} cells; at least 15%, 20%, 25%, 30%, 35% of the T_{CM} or T_{N/MEM} cells are CD4+ and at least 15%, 20%, 25%, 30%, 35% of the T_{CM} or T_{N/MEM} cells are CD8+ cells) transduced by a vector comprising an expression cassette encoding a chimeric antigen receptor, wherein chimeric antigen receptor comprises an amino acid sequence selected from SEQ ID NOs: 10, 1, 2, 4 and 5; comprises an amino acid sequence that is identical to an amino acid sequence selected from SEQ ID NO: 10, 1, 2, 4 and 5 except for the presence of no more than 5 amino acid (e.g., 4, 3, 2, 1) substitutions, deletions or insertions; comprises an amino acid sequence that is identical to an amino acid sequence selected from SEQ ID NOs: 10, 1, 2, 4 and 5 except for the presence of no more than 5 amino acid substitutions; and comprises an amino acid sequence that is identical to an amino acid sequence selected from SEQ ID NOs: 10, 1, 2, 4 and 5 except for the presence of no more than 2 amino acid substitutions.. In various embodiments: the population of human T cells comprise central memory T cells; the cancer is B cell leukemia or B cell lymphoma; and the transduced human T cells where prepared by a method comprising obtaining T cells from the patient, treating the T cells to isolate central memory T cells, and transducing at least a portion of the central memory cells to with a viral vector comprising an expression cassette encoding a chimeric antigen receptor, wherein chimeric antigen receptor comprises an amino acid sequence selected from SEQ ID NOs: 10, 1, 2, 4 and 5; comprises an amino acid sequence that is identical to an amino acid sequence selected from SEQ ID NO: 10, 1, 2, 4 and 5 except for the presence of no more than 5 amino acid (e.g., 4, 3, 2, 1) substitutions, deletions or insertions; comprises an amino acid sequence that is identical to an amino acid sequence selected from SEQ ID NOs: 10, 1, 2, 4 and 5 except for the presence of no more than 5 amino acid substitutions; and comprises an amino acid sequence that is identical to an amino acid sequence selected from SEQ ID NOs: 10, 1, 2, 4 and 5 except for the presence of no more than 2 amino acid substitutions.

Also described are method is which the T cells administered to the patient harbor a nucleic acid molecule encoding an polypeptide comprising an amino acid sequence that is at least 95% identical to an amino acid sequence selected from and SEQ ID NOs: 10, 1, 2, 4 and 5; a nucleic acid molecule encoding an polypeptide comprising an amino acid sequence that is identical to an amino acid sequence selected from SEQ ID NO: 10, 1, 2, 4 and 5 except for the presence of no more than 5 amino acid (e.g., 4, 3, 2, 1) substitutions, deletions or insertions; a nucleic acid molecule encoding an polypeptide comprising an amino acid sequence that is identical to an amino acid sequence selected from SEQ ID NOs: 10, 1, 2, 4 and 5 except for the presence of no more than 5 amino acid substitutions; and a nucleic acid molecule encoding an polypeptide comprising an amino acid sequence that is identical to an amino acid sequence selected from SEQ ID NOs: 10, 1, 2, 4 and 5 except for the presence of no more than 2 amino acid substitutions.

In some cases, the CD19 CAR comprises the amino acid sequence of SEQ ID NO: 10 and an amino acid sequence of an ScFv that binds human CD19.

In some cases: the T cells expressing a CD19 targeted CAR are T cells have been cultured in a culture medium having 60mg/dL glucose and 2.8 mEq/L potassium; the T cells expressing a CD19 targeted CAR are T cells have been activated by exposure to CD19 prior to administration; the T cells have been activated by exposure to cells expressing CD19 prior to administration; and the T cells cells have been cultured in a culture medium having 60mg/dL glucose and 2.8 mEq/L potassium and have been activated by exposure to CD19 prior to administration or by exposure to cells expressing CD19 prior to administration.

In some cases, the CD19 CAR comprises the amino acid sequence of any of SEQ ID Nos: 10, 10, 1, 2, 4 and 5 except for the presence of no more than 2 single amino acid substitutions.

The CAR described herein can include a spacer region located between the targeting domain and the transmembrane domain. A variety of different spacers can be used. Some of them include at least portion of a human Fc region, for example a hinge portion of a human Fc region or a CH3 domain or variants thereof. Table 1 below provides various spacers that can be used in the CARs described herein.

**Table 1: Examples of Spacers**

| Name | Length | Sequence |
|---|---|---|
| a3 | 3 aa | AAA |
| linker | 10 aa | GGGSSGGGSG (SEQ ID NO:11) |
| IgG4 hinge (S→P) (S228P) | 12 aa | ESKYGPPCPPCP (SEQ ID NO:12) |
| IgG4 hinge | 12 aa | ESKYGPPCPSCP (SEQ ID NO:13) |
| IgG4 hinge + linker | 22 aa | ESKYGPPCPPCPGGGSSGGGSG (SEQ ID NO:14) |
| CD28 hinge | 39 aa | IEVMYPPPYLDNEKSNGTIIHVKGKHLCPSPLFPGPS KP (SEQ ID NO:15) |
| CD8 hinge-48aa | 48 aa | AKPTTTPAPRPPTPAPTIASQPLSLRPEACRPAAGGA VHTRGLDFACD (SEQ ID NO:16) |
| CD8 hinge-45aa | 45aa | TTTPAPRPPTPAPTIASQPLSLRPEACRPAAGGAVHT RGLDFACD (SEQ ID NO:17) |
| IgG4(HL-CH3) | 129 aa | ESKYGPPCPPCPGGGSSGGGSGGQPREPQVYTLPPS QEEMTKNQVSLTCLVKGFYPSDIAVEWESNGQPEN NYKTTPPVLDSDGSFFLYSRLTVDKSRWQEGNVFS CSVMHEALHNHYTQKSLSLSLGK (SEQ ID NO:18) |
| IgG4(L235E,N297Q) | 229 aa | ESKYGPPCPSCPAPEFEGGPSVFLFPPKPKDTLMISR TPEVTCVVVDVSQEDPEVQFNWYVDGVEVHQAKT KPREEQFNSTYRVVSVLTVLHQDWLNGKEYKCKV SNKGLPSSIEKTISKAKGQPREPQVYTLPPSQEEMT KNQVSLTCLVKGFYPSDIAVEWESNGQPENNYKTT PPVLDSDGSFFLYSRLTVDKSRWQEGNVFSCSVMH EALHNHYTQKSLSLSLGK (SEQ ID NO:19) |
| IgG4(S228P, L235E,N297Q) | 229 aa | ESKYGPPCPPCPAPEFEGGPSVFLFPPKPKDTLMISR TPEVTCVVVDVSQEDPEVQFNWYVDGVEVHQAKT KPREEQFNSTYRVVSVLTVLHQDWLNGKEYKCKV SNKGLPSSIEKTISKAKGQPREPQVYTLPPSQEEMT KNQVSLTCLVKGFYPSDIAVEWESNGQPENNYKTT PPVLDSDGSFFLYSRLTVDKSRWQEGNVFSCSVMH EALHNHYTQKSLSLSLGK (SEQ ID NO:20) |
| IgG4(CH3) | 107 aa | GQPREPQVYTLPPSQEEMTKNQVSLTCLVKGFYPS DIAVEWESNGQPENNYKTTPPVLDSDGSFFLYSRLT VDKSRWQEGNVFSCSVMHEALHNHYTQKSLSLSL GK (SEQ ID NO:21) |

Some spacer regions include all or part of an immunoglobulin (e.g., IgG1, IgG2, IgG3, IgG4) hinge region, i.e., the sequence that falls between the CH1 and CH2 domains of an immunoglobulin, e.g., an IgG4 Fc hinge or a CD8 hinge. Some spacer regions include an immunoglobulin CH3 domain or both a CH3 domain and a CH2 domain. The immunoglobulin derived sequence can include one or more amino acid modifications, for example, 1, 2, 3, 4 or 5 substitutions, e.g., substitutions that reduce off-target binding.

An "amino acid modification" refers to an amino acid substitution, insertion, and/or deletion in a protein or peptide sequence. An "amino acid substitution" or "substitution" refers to replacement of an amino acid at a particular position in a parent peptide or protein sequence with another amino acid. A substitution can be made to change an amino acid in the resulting protein in a non-conservative manner (i.e., by changing the codon from an amino acid belonging to a grouping of amino acids having a particular size or characteristic to an amino acid belonging to another grouping) or in a conservative manner (i.e., by changing the codon from an amino acid belonging to a grouping of amino acids having a particular size or characteristic to an amino acid belonging to the same grouping). Such a conservative change generally leads to less change in the structure and function of the resulting protein. The following are examples of various groupings of amino acids: 1) Amino acids with nonpolar R groups: Alanine, Valine, Leucine, Isoleucine, Proline, Phenylalanine, Tryptophan, Methionine; 2) Amino acids with uncharged polar R groups: Glycine, Serine, Threonine, Cysteine, Tyrosine, Asparagine, Glutamine; 3) Amino acids with charged polar R groups (negatively charged at pH 6.0): Aspartic acid, Glutamic acid; 4) Basic amino acids (positively charged at pH 6.0): Lysine, Arginine, Histidine (at pH 6.0). Another grouping may be those amino acids with phenyl groups: Phenylalanine, Tryptophan, and Tyrosine.

A variety of transmembrane domains can be used in CAR expressed by the cells used in the methods described herein. Table 2 includes examples of suitable transmembrane domains. Where a spacer region is present, the transmembrane domain is located carboxy terminal to the spacer region.

**Table 2: Examples of Transmembrane Domains**

| **Name** | **Accession** | **Length** | **Sequence** |
|---|---|---|---|
| CD3z | J04132.1 | 21 aa | LCYLLDGILFIYGVILTALFL (SEQ ID NO:22) |
| CD28 | NM_006139 | 27aa | FWVLVVVGGVLACYSLLVTVAFIIFWV (SEQ ID NO:23) |
| CD28(M) | NM_006139 | 28aa | MFWVLVVVGGVLACYSLLVTVAFIIFWV (SEQ ID NO:24) |
| CD4 | M35160 | 22aa | MALIVLGGVAGLLLFIGLGIFF (SEQ ID NO:25) |
| CD8tm | NM_001768 | 21aa | IYIWAPLAGTCGVLLLSLVIT (SEQ ID NO:26) |
| CD8tm2 | NM_001768 | 23aa | IYIWAPLAGTCGVLLLSLVITLY (SEQ ID NO:27) |
| CD8tm3 | NM_001768 | 24aa | IYIWAPLAGTCGVLLLSLVITLYC (SEQ ID NO:28) |
| 41BB | NM_001561 | 27aa | IISFFLALTSTALLFLLFF LTLRFSVV (SEQ ID NO:29) |

Many of the CAR expressed by the cells used in the methods described herein include one or more (e.g., two) costimulatory domains. The costimulatory domain(s) are located between the transmembrane domain and the CD3ζ signaling domain. Table 3 includes examples of suitable costimulatory domains together with the sequence of the CD3ζ signaling domain.

**Table 3: CD3ζ Signaling Domain and Examples of Costimulatory Domains**

| **Name** | **Accession** | **Length** | **Sequence** |
|---|---|---|---|
| CD3ζ | J04132.1 | 113 aa | RVKFSRSADAPAYQQGQNQLYNELNLGRREEYD VLDKRRGRDPEMGGKPRRKNPQEGLYNELQKDK MAEAYSEIGMKGERRRGKGHDGLYQGLSTATKD TYDALHMQALPPR (SEQ ID NO: 30) |
| CD28 | NM_006139 | 42aa | RSKRSRLLHSDYMNMTPRRPGPTRKHYQPYAPPR DFAAYRS (SEQ ID NO: 31) |
| CD28gg* | NM_006139 | 42aa | RSKRSRGGHSDYMNMTPRRPGPTRKHYQPYAPP RDFAAYRS (SEQ ID NO:32) |
| 41BB | NM_001561 | 42 aa | KRGRKKLLYIFKQPFMRPVQTTQEEDGCSCRFPEE EEGGCEL (SEQ ID NO:33) |
| OX40 | | 42 aa | ALYLLRRDQRLPPDAHKPPGGGSFRTPIQEEQADA HSTLAKI (SEQ ID NO:34) |

### DESCRIPTION OF DRAWINGS

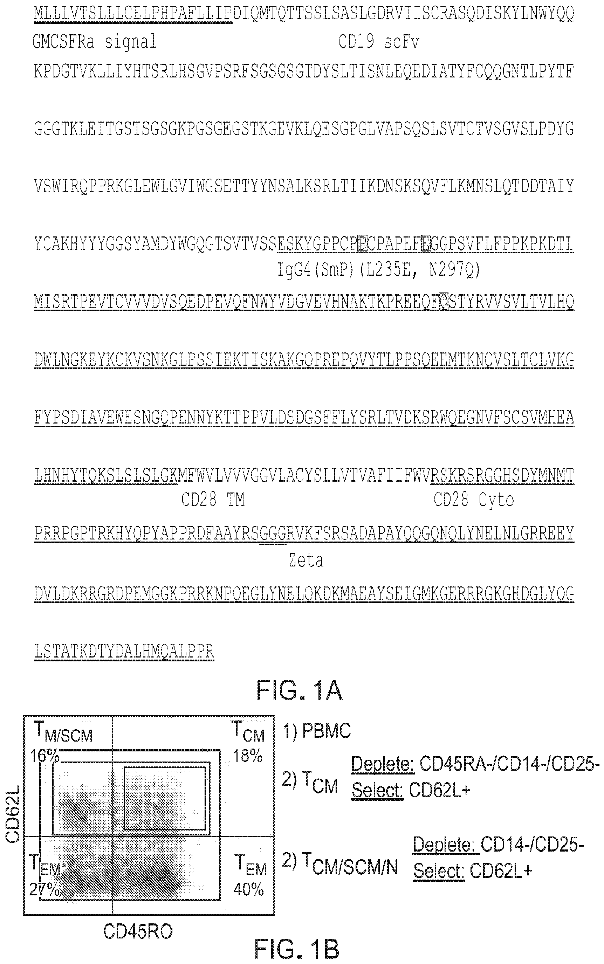

**FIG 1****: Amino acid sequence of** a **CD19 CAR with** a **Signal Sequence.** SEQ ID NO: 1 is the sequence of the CD19 CAR (including the signal sequence; GMCSFRa signal peptide (SEQ ID NO: 9)) used in the studies described herein. The various domains are labelled and indicated by underlining or lack of underling with the exception of the GGG linker which is indicated by a double underline. (**A**). Example of selected surface marker expression profiles for PBMC, T_{CM}, and T_{SCM/CM/N} **(B)**.

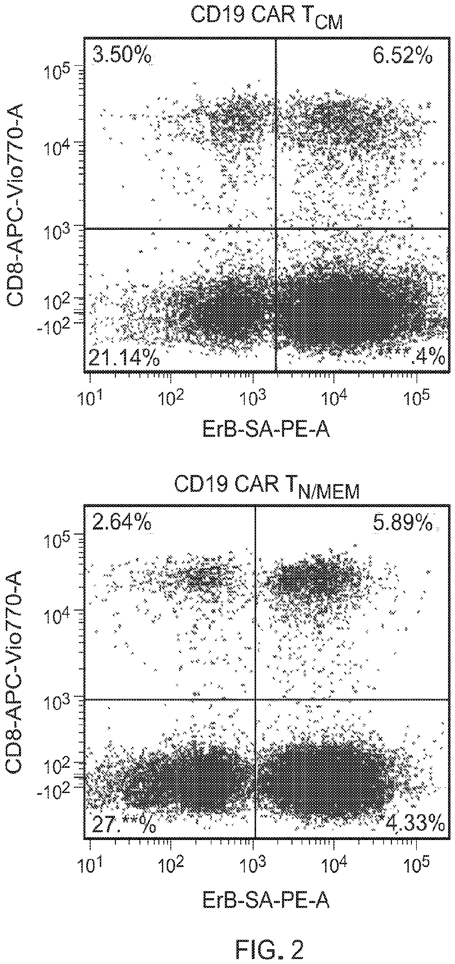

**FIG 2****: Phenotype of T_{CM} and T_{N/MEM}-derived CD19CAR T cells**. T_{CM} (CD62L+CD45RO+) and T_{N/MEM} (CD62L+) cells were isolated from healthy donor PBMCs and transduced to express a second generation CD19 specific chimeric antigen receptor (CAR) with a truncated EGFR (EGFRt). CAR T cells were expanded in the presence of IL-2 50U/mL and IL-15 0.5ng/mL for 14 days. CAR expression was defined by Erbitux-biotin and streptavidin (SA) staining.

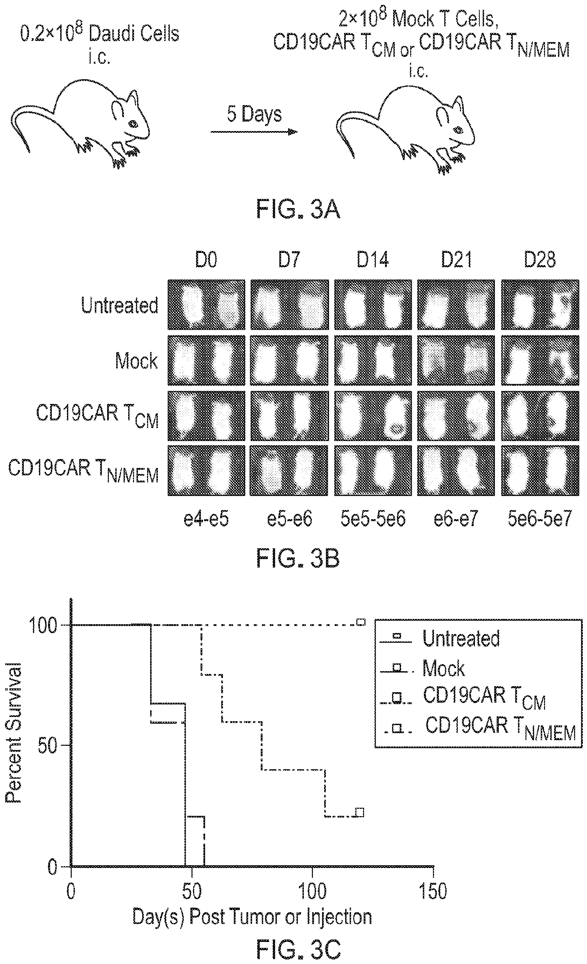

**FIG 3****: T_{N/MEM}-derived CD19CAR T cells exhibit superior anti-CNSL activity to T_{CM}**-**derived CD19CAR T cells.** (**A**) NSG mice were injected intracranially (IC) with 0.2×10⁶GFP+ ffluc+ Daudi cells. Five days later, mice received an IC local infusion of 2×10⁶ untransduced mock T cells, T_{CM} or T_{N/MEM}-derived CD19 CAR T cells. (**B**) Tumor signals were monitored by bioluminescent imaging once a week. (C) Kaplan-Meier survival curves are presented. N=5.

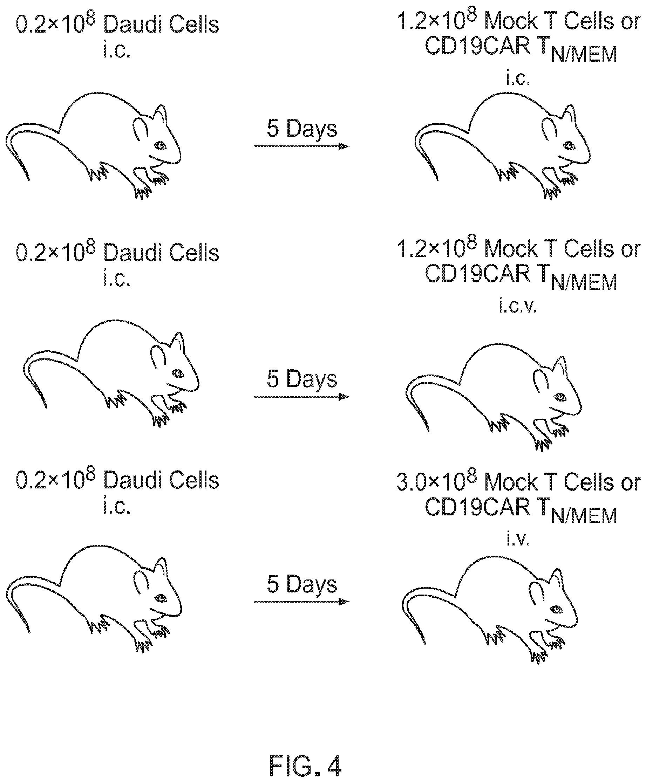

**FIG 4**: **Comparing efficacy of T_{N/MEM}-derived CD19CAR T cells against CNSL via three different delivery routes.** NSG mice were injected intracranially (IC) with 0.2×10⁶ GFP+ ffluc+ Daudi cells. Tumor was allowed to engraft for five days before mice received T_{N/MEM}-derived CD19 CAR T cells via three different delivery routes: 1) intracranial (IC) local infusion with 1.2×10⁶ CD19 CAR T cells, 2) intracerebroventricular (ICV) administration with 1.2×10⁶ CD19 CAR, and 3) intravenous (IV) injection with 3×10⁶ CD19 CAR T cells. Tumor growth was evaluated by bioluminescent imaging once a week.

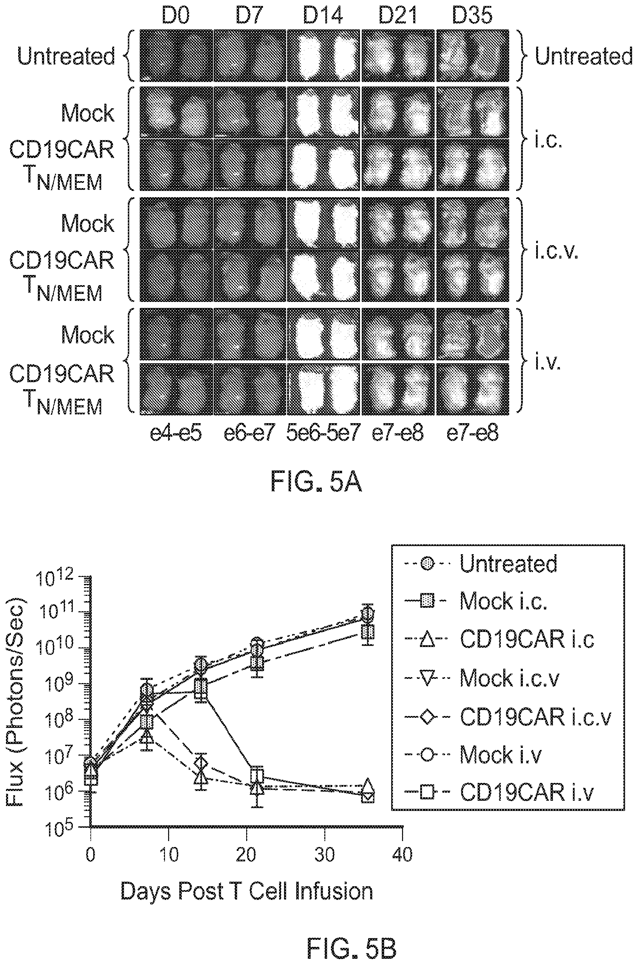

**FIG 5****: Administration of T_{N/MEM}-derived CD19CAR T cells by all routes is able to eradicate CNSL.** Both IC and ICV delivery of CD19 CAR T cells were able to completely eradicate CNS lymphoma in all mice by day 14 post CAR T cell infusion. Intravenous (IV) infusion induced significant antitumor activity 21 days post T cell infusion.

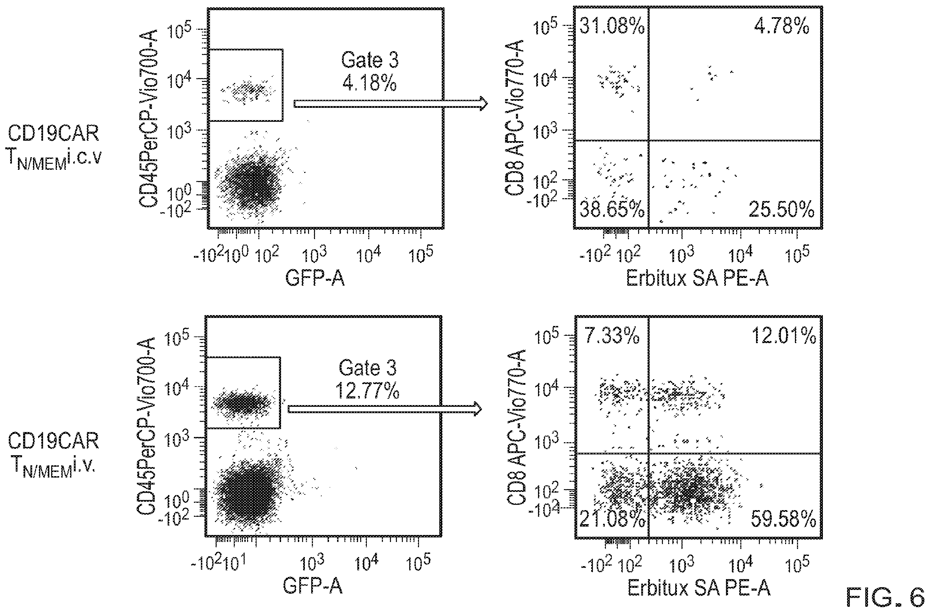

**FIG 6****: CD19CAR T cells are detected in the peripheral blood of IV and ICV treated mice.** 28 days after CAR T cell infusion, peripheral blood was collected retro-orbitally. Percentages of CAR T cells (CD45+ Erbitux+) are depicted.

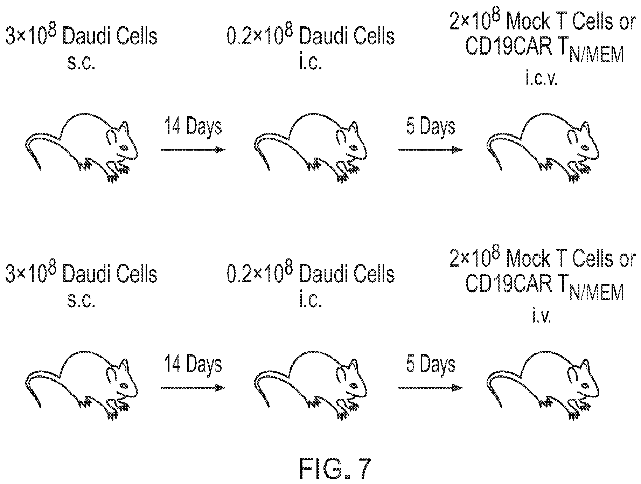

**FIG 7****: Comparison of T_{N/MEM}-derived CD19 CAR T cells administered IV and ICV.** NSG mice were injected subcutaneously (SC) with 3×10⁶ GFP+ ffluc+ Daudi cells and 14 days later intracranially (IC) with 0.2×10⁶ Daudi cells. Five days following IC tumor inoculation, 2×10⁶ untransduced mock T cells or T_{N/MEM}-derived CD19 CAR T cells were administered either ICV or IV.

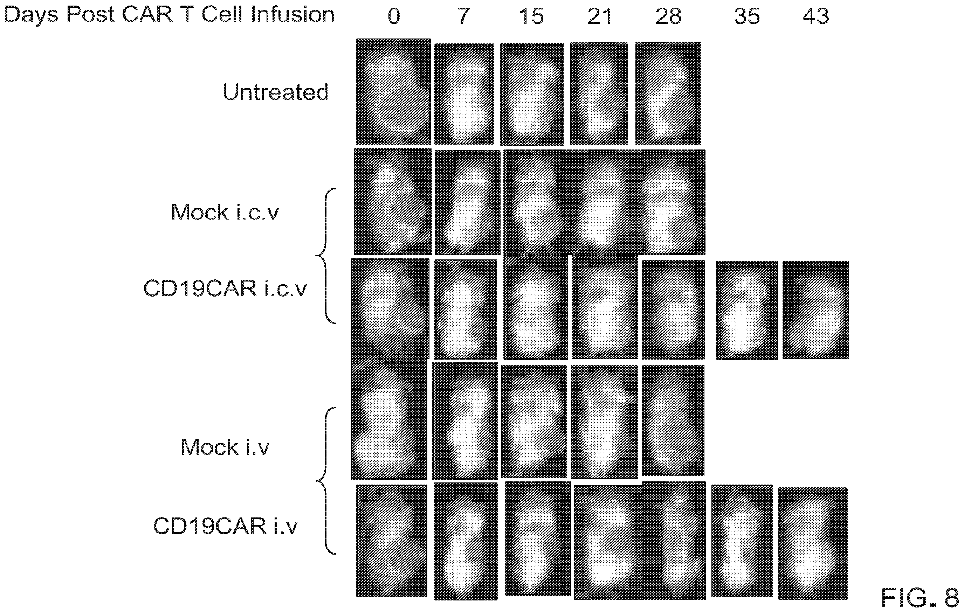

**FIG 8****: T_{N/MEM}-derived CD19CAR T cells administered ICV are more efficacious than IV delivered CAR T cells in controlling both CNSL and systemic lymphoma.** Both brain and flank tumors show faster regression in mice treated with CD19 CAR T cells administered ICV as compared to those treated with IV delivered CD19 CAR T cells.

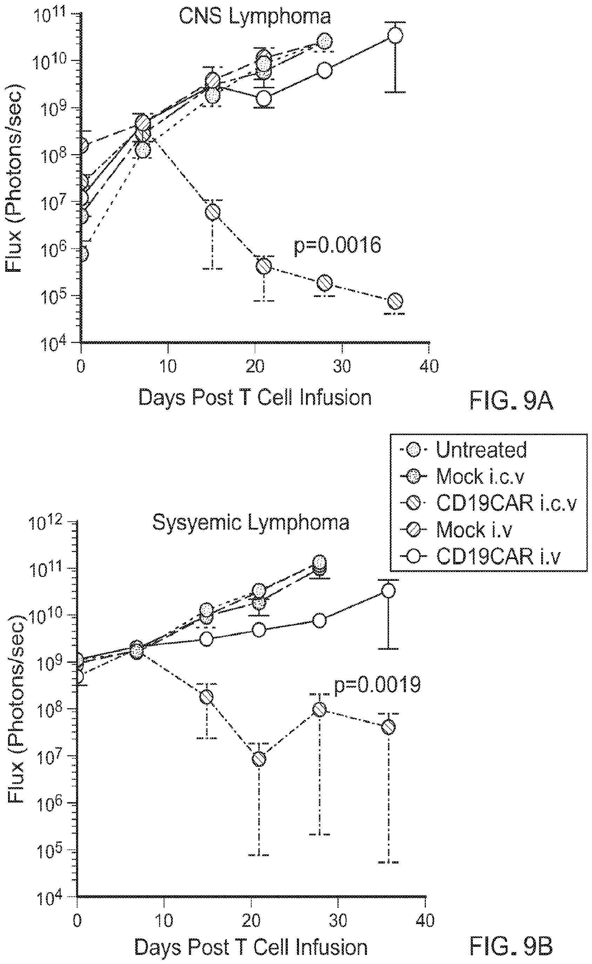

**FIG 9****: T_{N/MEM}-derived CD19CAR T cells administered ICV are more efficacious than IV delivered CAR T cells in controlling both CNSL and systemic lymphoma.** CD19+fflu+ tumor burden was monitored weekly. Flux (photons/sec) was determined by measuring bioluminescence with imaging once a week. CD19 CAR T cells administered ICV demonstrate the greatest ability to target both systemic (**A**) and CNS lymphoma (**B**).

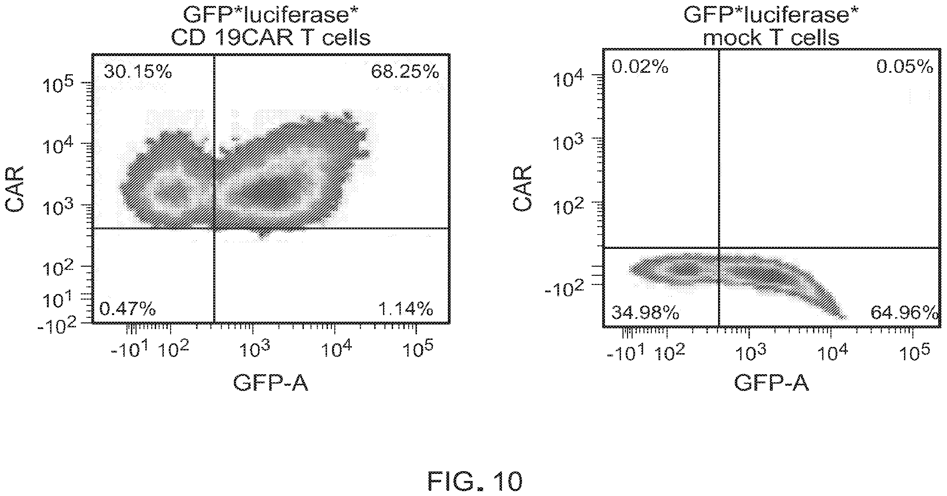

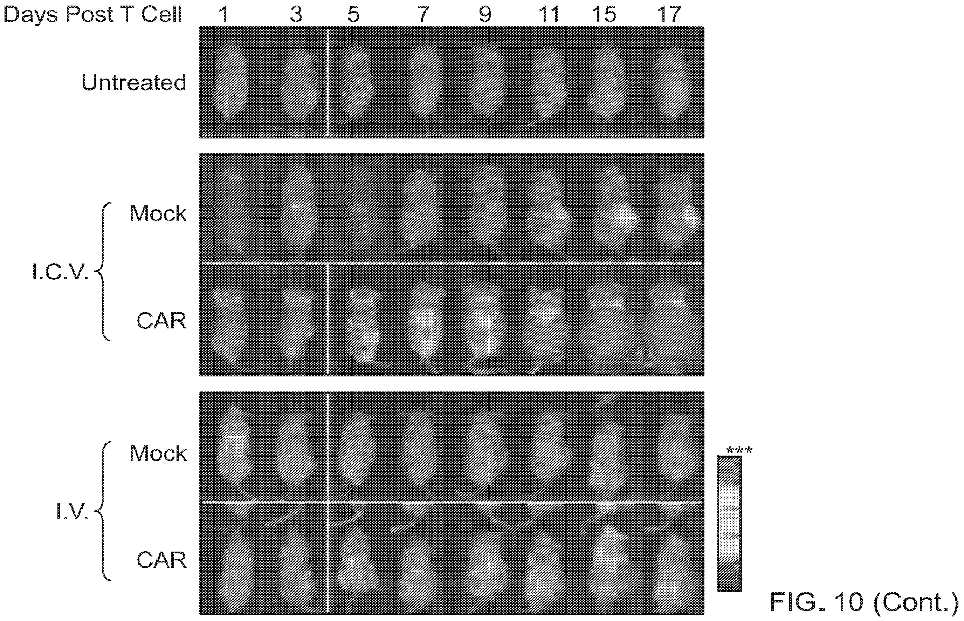

**FIG 10**: **ICV administered T_{N/MEM}-derived CD19CAR T cells exhibited greater proliferative capacity than IV delivered ones in lymphoma mice.** CD19+fflu+ tumor was grafted on the right flank of the mice. Nineteen days after tumor engraftment, CAR T cells that were transduced with GFPffluc were administered via ICV or IV. CAR T cell proliferation was determined by measuring bioluminescence with imaging every other day.

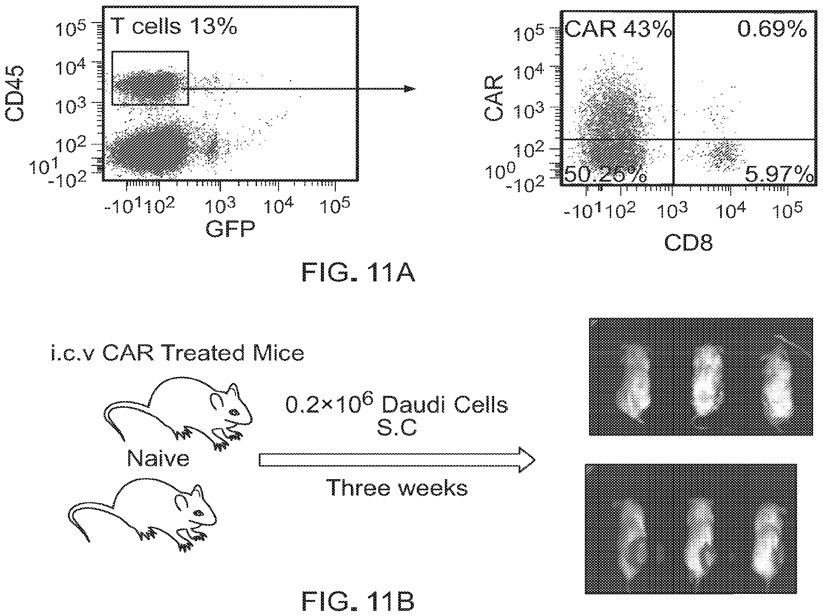

**FIG 11****: CD19 CAR T cells administered ICV persist long *in vivo* and maintain antitumor activity against tumor re-challenge.** Daudi lymphoma cells were engineered to express firefly luciferase and injected into mouse brain as CNS lymphoma model. After tumor engraftment, mice received 2×10⁶ CD19CAR T cells administered intracerebroventricularly. One hundred days post CAR T cell treatment, blood was collected from the mice and human T cells and CAR T cells were analyzed with flow cytometry (**A**). These mice were re-challenged by injecting new daudi tumor cells on the right flank of mice. Tumor growth was monitored by measuring bioluminescence with imaging (**B**).

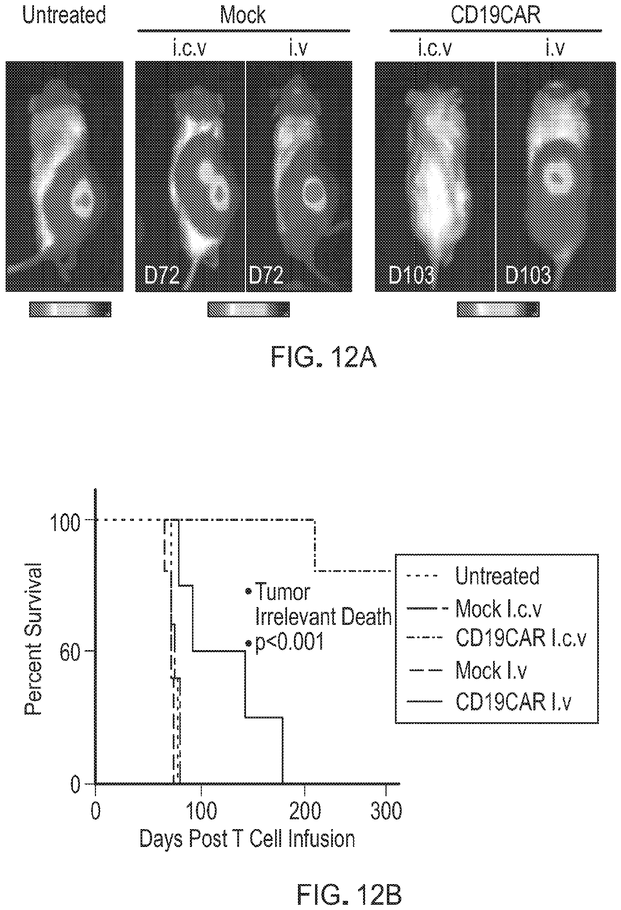

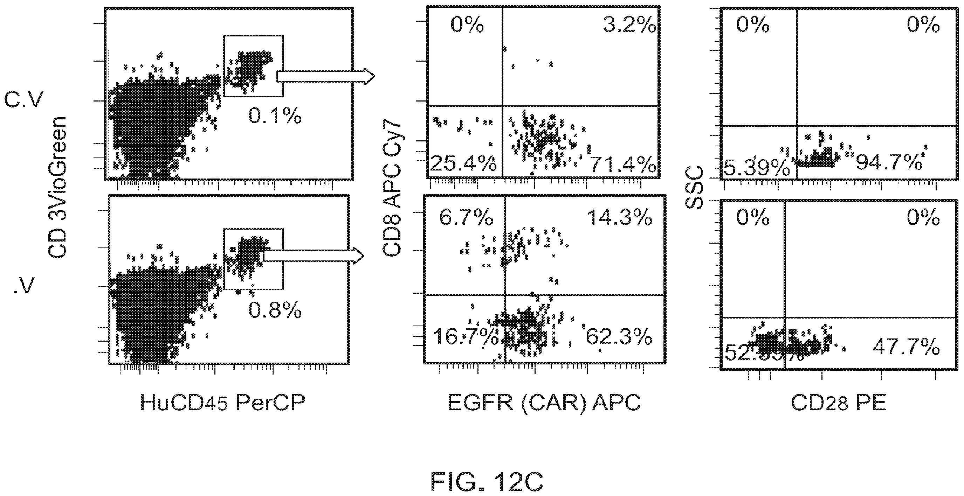

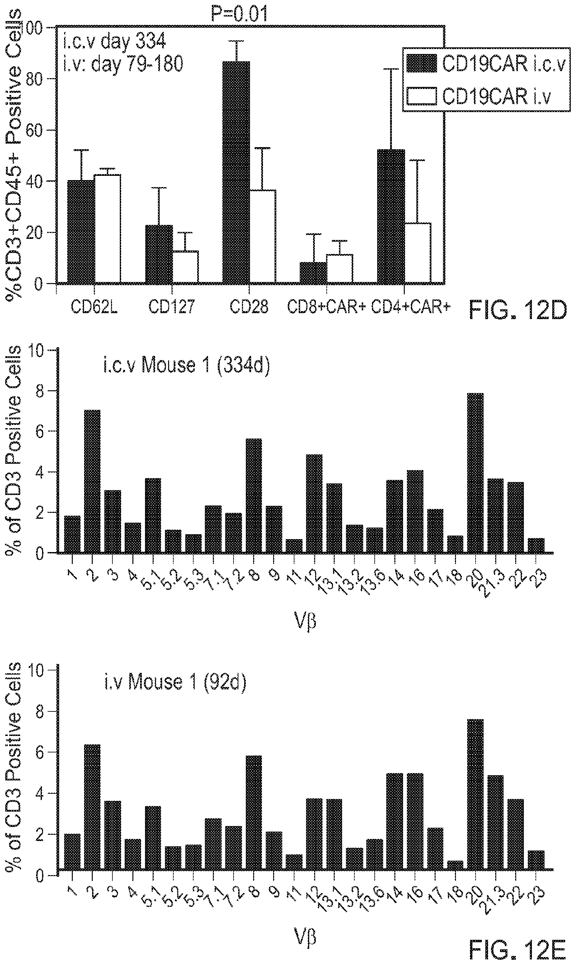

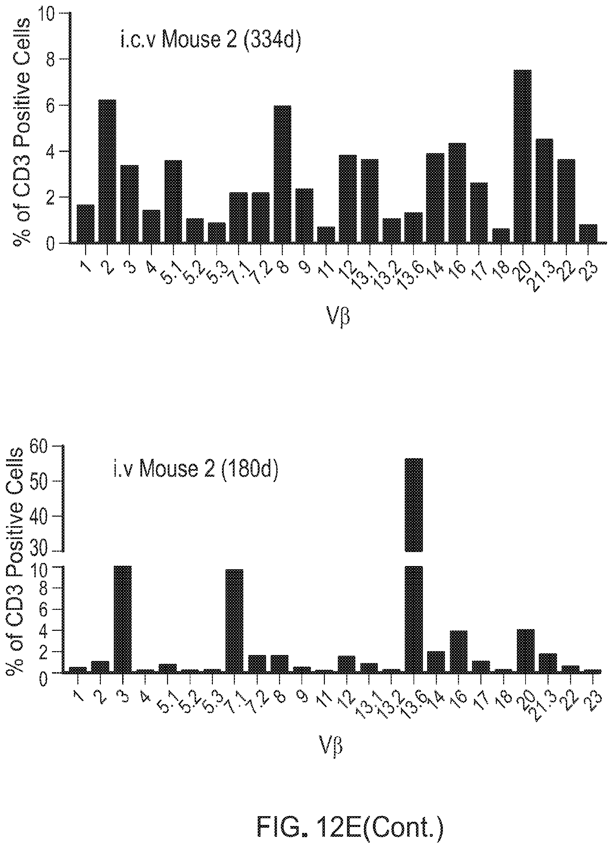

**FIG 12****: CD19-CAR T cells administered by ICV infusion are able to completely eradicate both CNS and systemic lymphoma.** NSG mice were injected subcutaneously (SC) with 3×10⁶ GFP+ ffluc+ Daudi cells and 14 days later intracranially (IC) with 0.2×10⁶ Daudi cells. Five days following IC tumor inoculation, 2×10⁶ untransduced mock T cells or CD19-CAR T cells were administered either ICV or IV Flux (photons/sec) was determined by measuring bioluminescence by live imaging once a week (**A**). N=5 mice per group. P-values were determined by comparing mean flux of each mock or CAR treated group with the untreated group using Kruskal-Wallis test. Log-rank (Mantel-Cox) test was used for survival analysis **(B).** Each *in vivo* experiment was repeated twice and representative data are presented. At the time of euthanization (day 334 for ICV group and up to 180 for IV group) blood was collected, stained with antibodies against human CD45, CD8, CD4, and CAR (Erbitux) and central memory T cell immune receptors, and analyzed by flow cytometry. Percentages of human T cells expressing CD62L, CD127, CD28, and CAR+ CD4 and CD8 T cell subsets are presented (mean± SEMs, N=3-4 mice per group). P value is depicted with two-way ANOVA analysis **(C-D).** Human T cells harvested from mouse spleens 334 day after ICV and 180 days post IV CAR T cell infusion were re-stimulated with REM method containing OKT3, irradiated PBMC, and LCL for 14 days and TCR repertoire was analyzed (**E**).

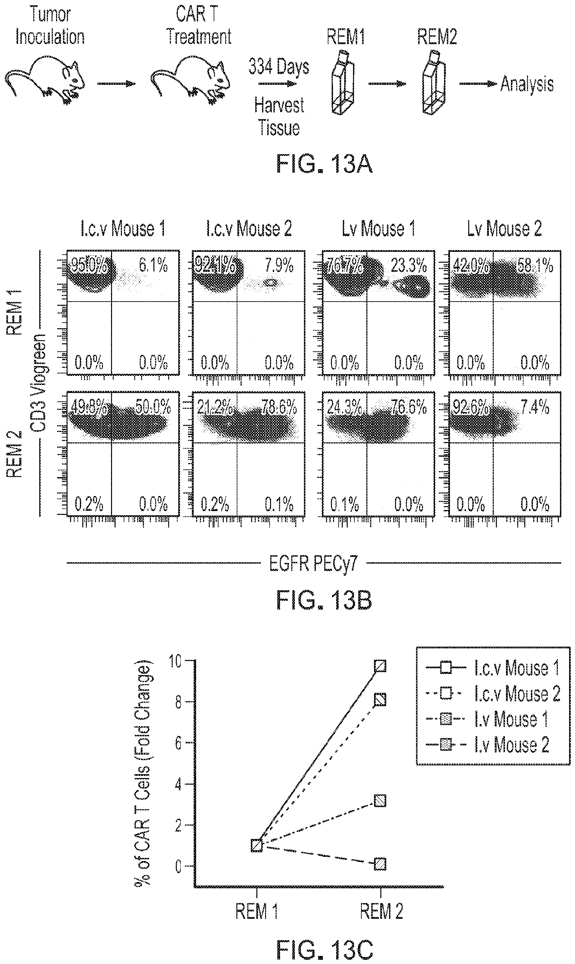

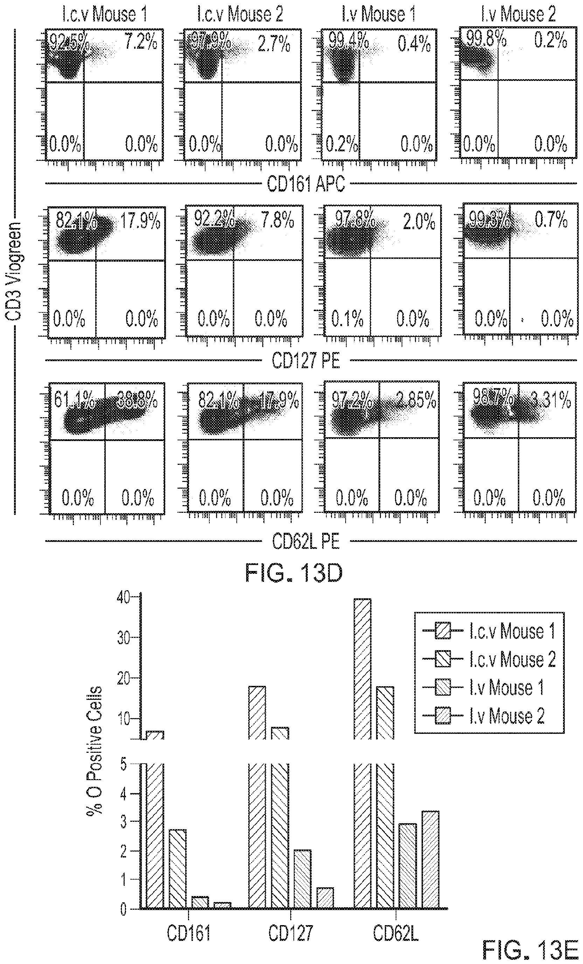

**FIG 13****: ICV administered CD19-CAR T cells exhibit greater proliferative capacity than IV delivered CD19-CAR T cells in tumor-bearing mice.** T cells harvested from mouse spleens (334 days for ICV mice and 180 days for IV mice) were stimulated with OKT3 and feeders, including PBMC and LCL, for 2 cycles (14 days/cycle) (A). Percentages of CAR T cells and fold changes post first and second stimulation are presented after staining with an antibody against EGFR **(B-C).** Phenotypic analysis for CD161, CD127, and CD62L expression was performed after the second stimulation **(D-E).**

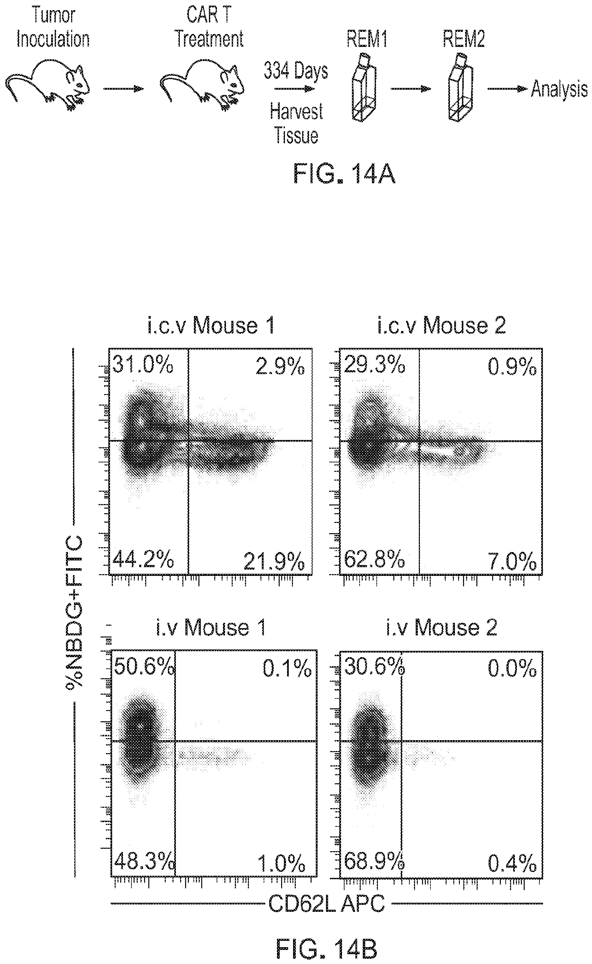

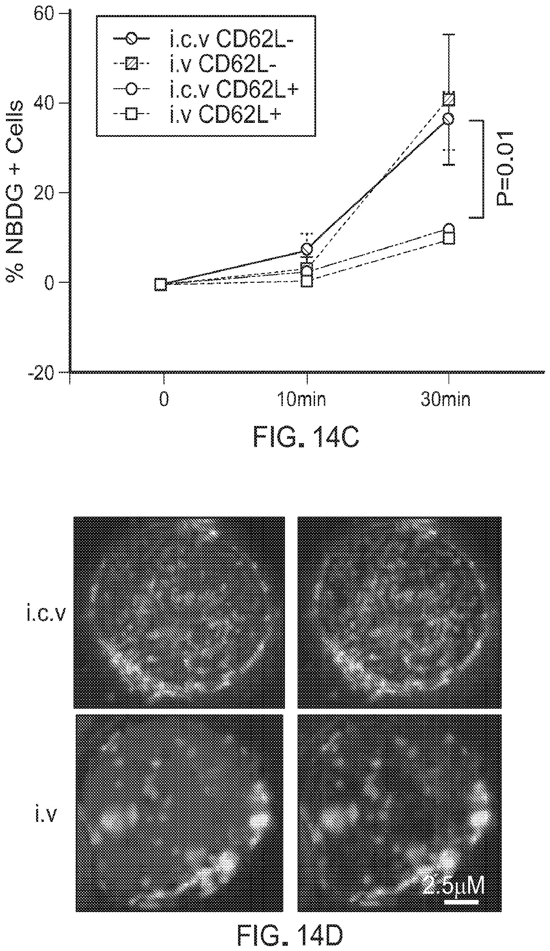

**FIG 14****: ICV and IV administered CD19-CAR T cells exhibit differences in glucose uptake and mitochondrial morphology.** T cells harvested from mice (334 days for ICV mice and 180 days for IV mice) were stimulated with OKT3 and feeders, including PBMC and LCL for 2 cycles (14 days/cycle) **(A).** Uptake of NBDG was analyzed after different time points. Data from the 30 min time point are presented (**B**). Percentages of 2-NBDG positive cells in CD62L positive and CD62L negative cell populations are presented (C). T Cells were incubated in T cell medium with 100 nM MitoTracker (Green) Mitochondrial selective probe for 30 mins at 37°C. 1×10⁵ cells were embedded onto slides by cytospin and fixed with 4% PFA before staining with DAPI for 1 min. The mitochondrial photos were taken using a LSM880 confocal microscope (**D**).

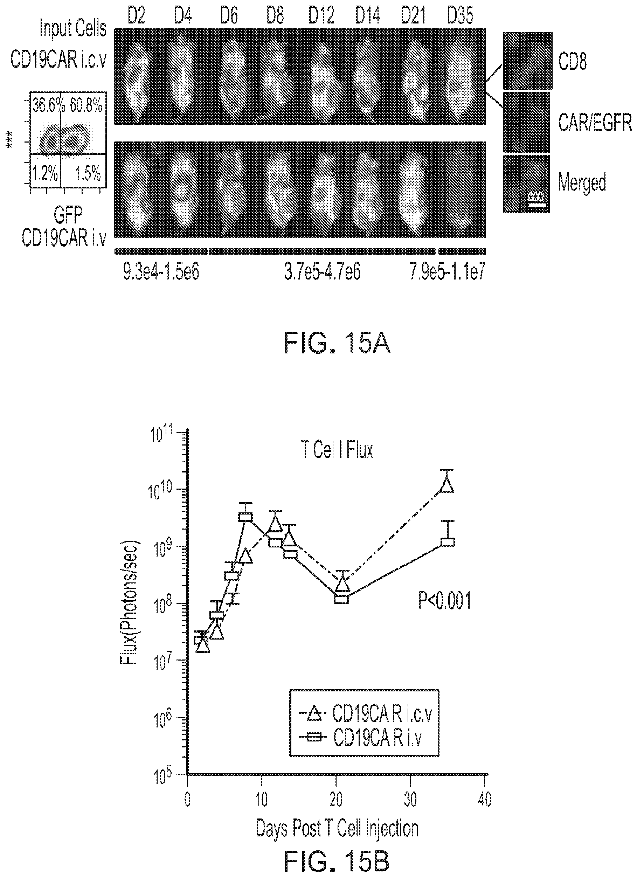

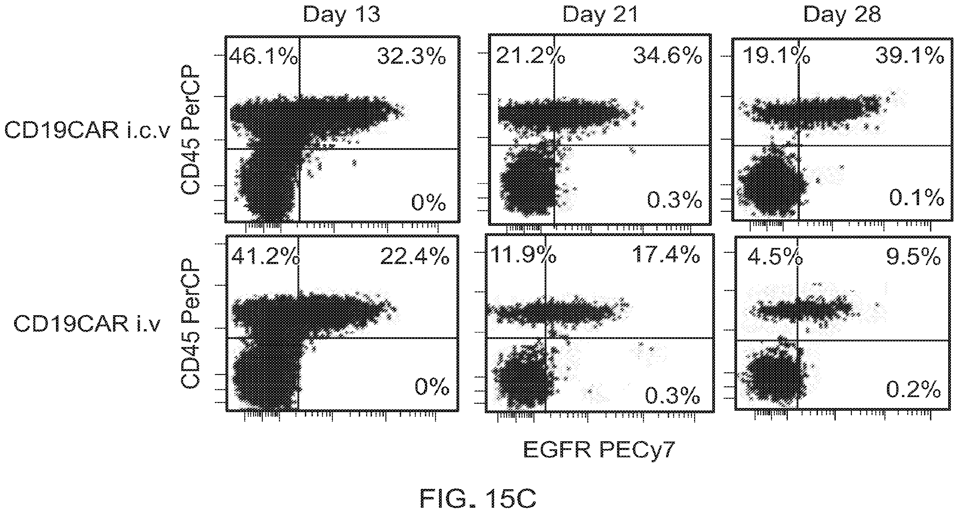

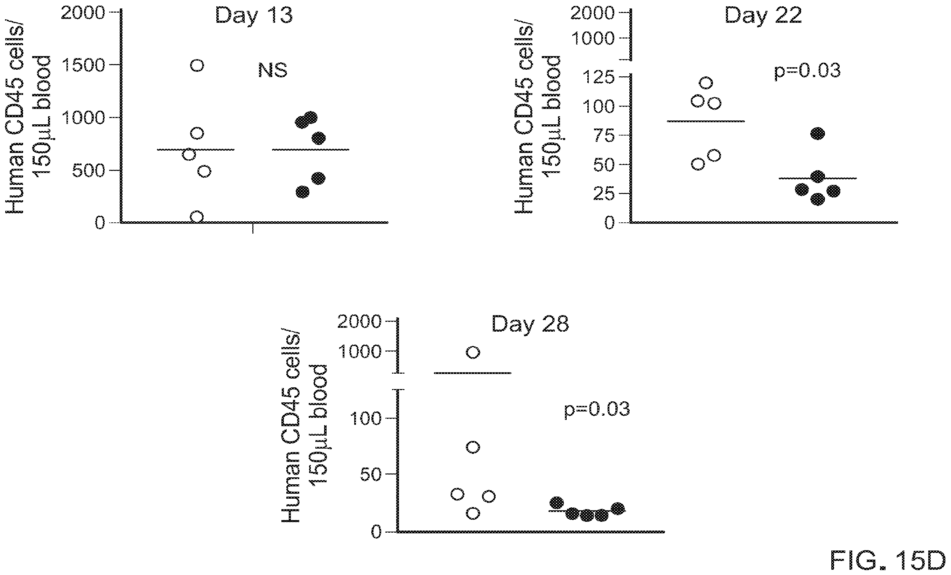

**FIG 15****: ICV delivered CAR T cells exhibit similar trafficking, but superior proliferative and persistence potential when compared with IV delivered CAR T cells.** CD19-CAR T cells were transduced with GFPffluc (**A**) and expanded in vitro. NSG mice were implanted with Daudi tumors (SC) on the right flank. Nineteen days after tumor engraftment, 2×10⁶ GFPffluc CAR T cells were administered via ICV or IV and CAR T cell proliferation was determined by measuring bioluminescence by live imaging every other day (**A-B**). The tumors were harvested 4 weeks after T cell infusion and stained for both CD3 and CAR (EGFR) using standard IHC (A). Blood was collected at different time points after CAR infusion and T cell (anti-human CD45) and CAR+ T cell (anti-EGFR) levels were detected by flow cytometry (**C-D**). N=5 mice per group. P-values were determined with the **Mann-Whitney test.** Experiments were repeated twice and representative data are presented.

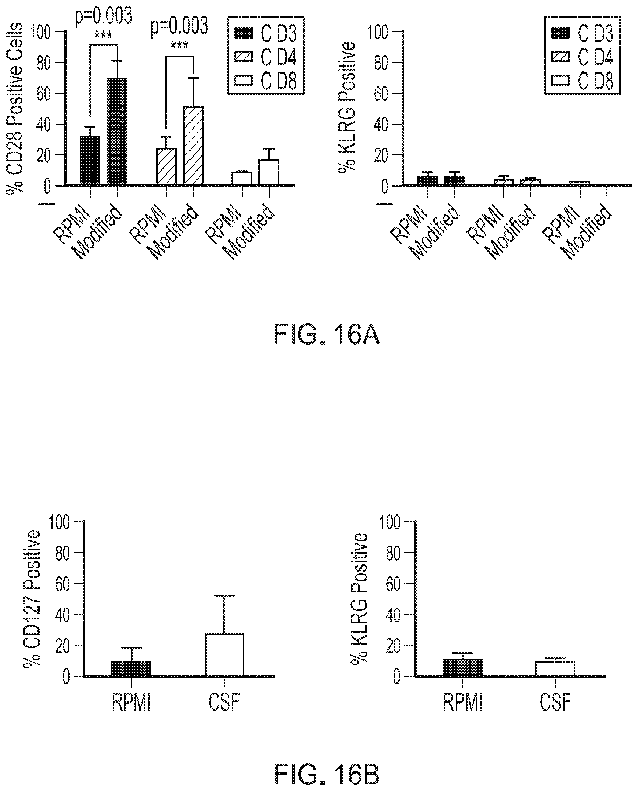

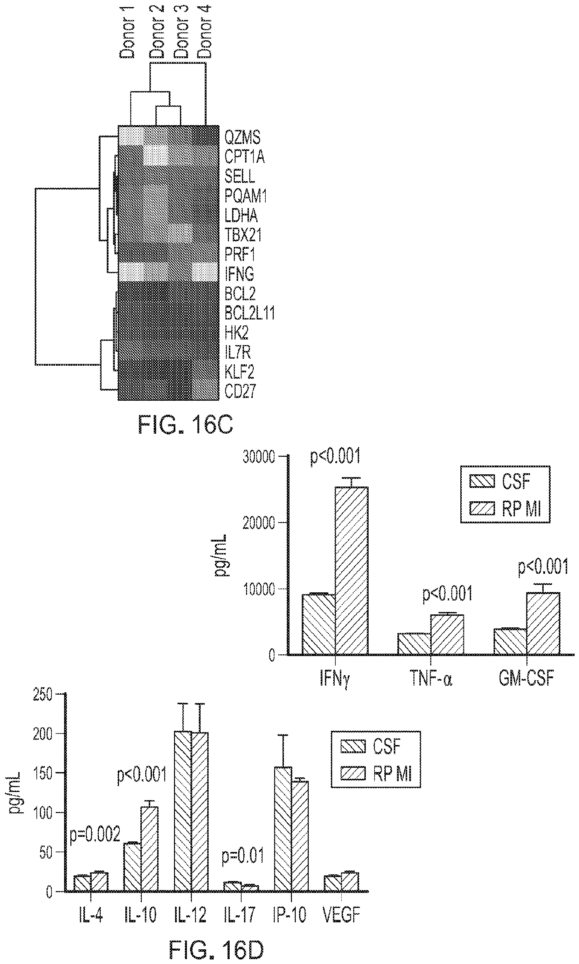

**FIG 16****: CSF environment-reprogrammed CAR T cells exhibit enhanced memory features and downregulated glycolytic metabolism.** CAR T cells were activated, transduced, and expanded for 13 days in modified RPMI (60mg/dL glucose and 2.8 mEq/L potassium) or in regular RPMI (200 mg/dL glucose and 5.3 mEq/L potassium). Phenotypic analysis was performed by flow cytometry. Data from 4 different human donors are presented. (P<0.01 with two-way ANOVA) **(A).** CD19-CAR T cells were co-cultured in RPMI and human CSF for 48 hrs. Phenotypic analysis was performed by flow cytometry. Means ± SEM of 4 different donors are presented (**B**). RT-PCR array with the CD19-CAR T cells co-cultured in RPMI and CSF for 48 hrs was performed. Fold changes of genes in CSF over RPMI >1.3 are listed (N=4 donors) **(C).** CAR T cells were stimulated with Daudi cells in CSF and RPMI for 48 hrs overnight. Cytokines in the supernatant were measured with cytometric bead array using the Bio-Plex Human Cytokine 17-Plex Panel **(D).** N=6 replicates. Two-way ANOVA was used for statistical analysis.

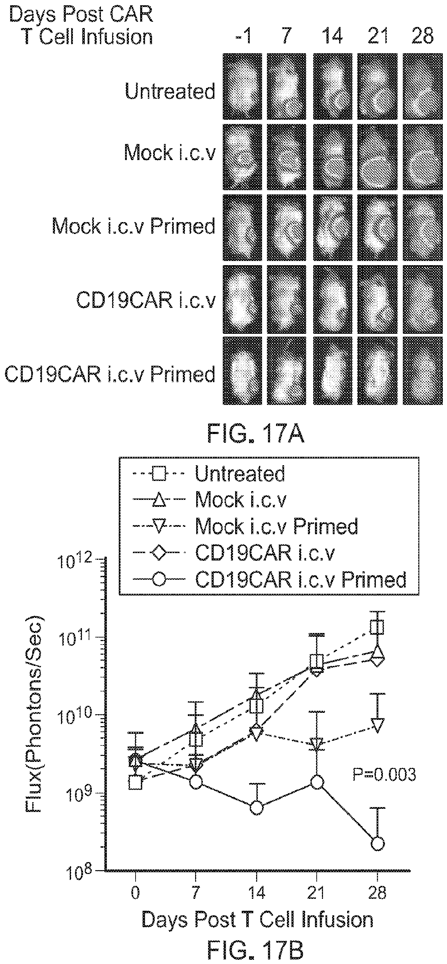

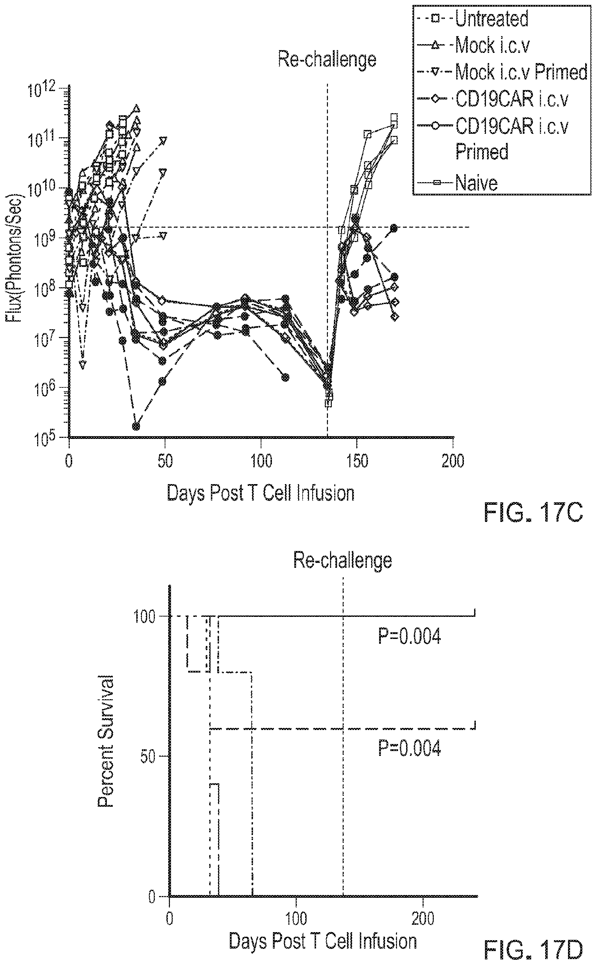

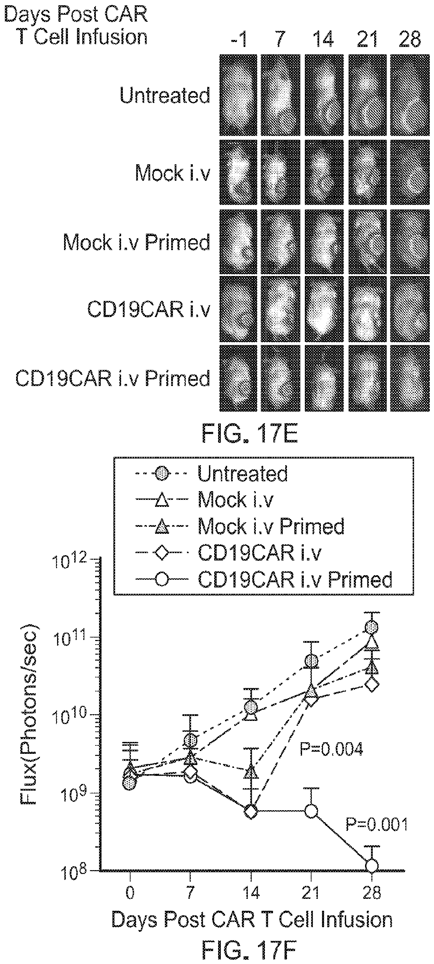

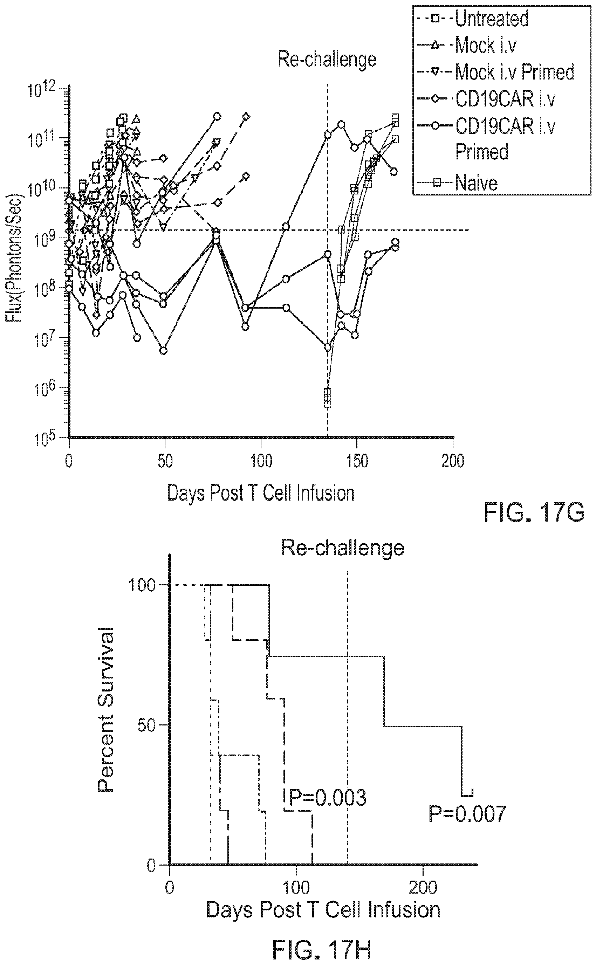

**FIG 17****: Antigen primed CAR T cells possess better effector and memory function and are able to resist tumor re-challenge.** NSG mice were inoculated with SC injections of 3×10⁶ Daudi cells on the right flank 19 days prior to CAR T cell treatment. On the day of CAR T cell infusion, 2×10⁶ CD19-CAR T cells were primed with irradiated Daudi cells at a 10:1 (CAR:tumor) ratio for 1 hr and then infused into tumor bearing mice through ICV (A-D) and IV (**E-H**). Mock T cells were used as controls. CD19+fflu+ tumor burden was monitored weekly. Flux (photons/sec) was determined by measuring bioluminescence by live imaging once a week. 135 days post initial CAR T treatment, mice were re-challenged with live tumor cells by SC injection. Naive mice were added as controls. Survival analysis was performed. N=5 mice per group. Log-rank (Mantel-Cox) and Kruskal-Wallis tests were used to ascertain the statistical significance.

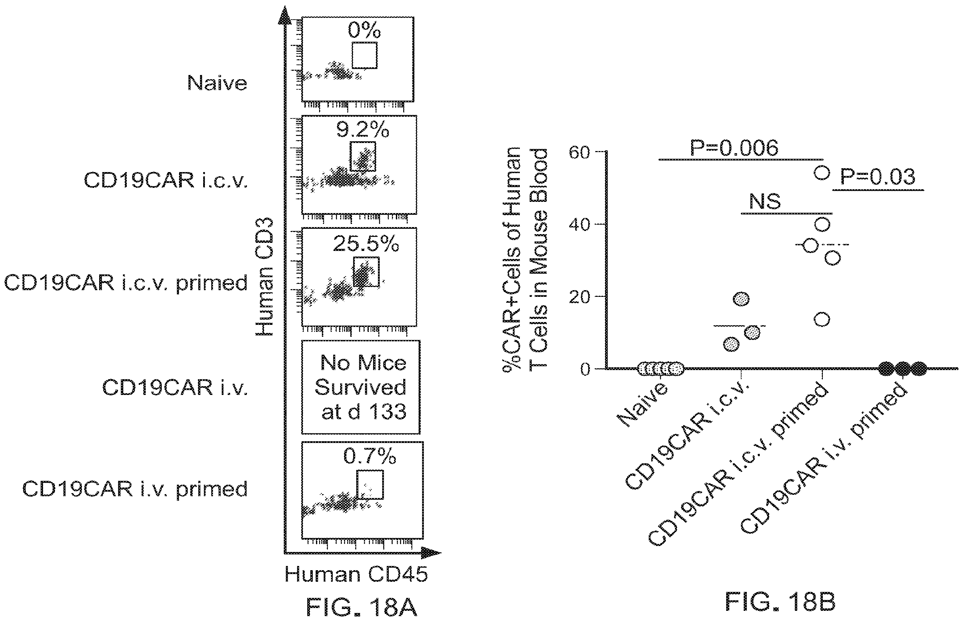

**FIG 18****: Antigen primed CAR T cells persist better than unprimed CAR T cells.** NSG mice were inoculated with 3×10⁶ Daudi cells SC on the right flank 19 days prior to CAR T cell treatment. On the day of CAR T cell infusion, 2×10⁶ CD19-CAR T cells were primed with irradiated Daudi cells at a 10:1 (CAR:tumor) ratio for 1 hr and then infused into tumor bearing mice through ICV and IV injection. Mock T cells were used as controls. 133 days post CAR T treatment, blood was collected retro orbitally and analyzed by flow cytometry after staining with antibodies against human CD45 and CAR (EGFR). The **Mann-Whitney** test was used to ascertain statistical significance (**A-B**).

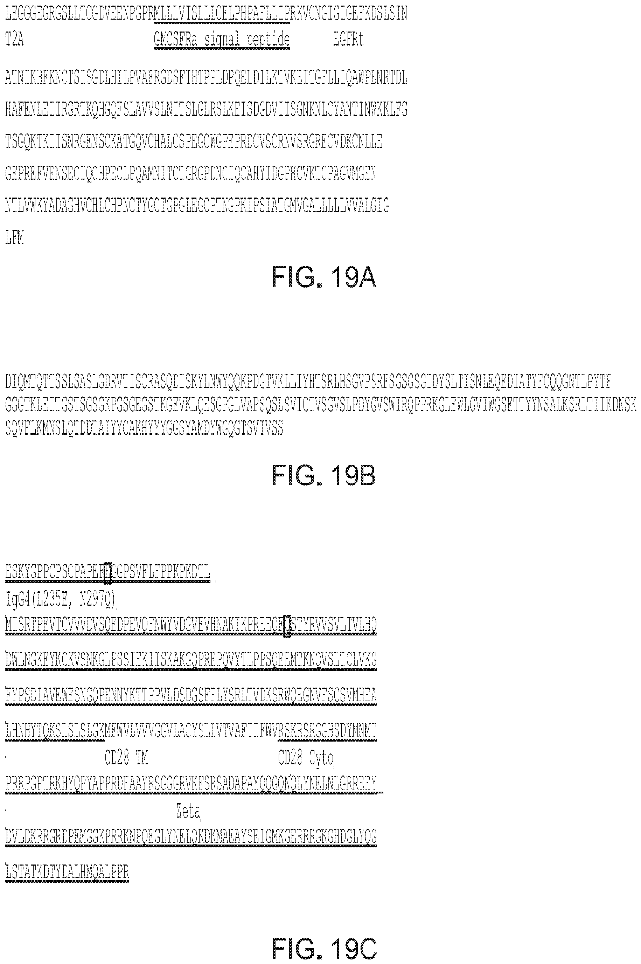

**FIG 19****: Amino Acid Sequence of a Marker and a CD19 scFv.** (A) SEQ ID NO:6 is the sequence of a marker that can follow the sequence of a CD19 CAR. It includes a T2A skip sequence (SEQ ID NO: 7) and a modified EGFR that lacks signaling activity (SEQ ID NO:8) as well as a GMCSFRa signal peptide (SEQ ID NO: 9) and can follow an CAR sequence to provide a useful marker that is expressed separately from the mature CAR as a surface maker and by the same cell. **(B)** Sequence of scFv used in the CD19 CAR described herein. (**C**) Sequence of the CD19 CAR used in the studies described herein without the CD19 scFv sequence (SEQ ID NO:10). This sequence can be joined to any suitable CD19 scFv sequence to create an alternative CD19 CAR.

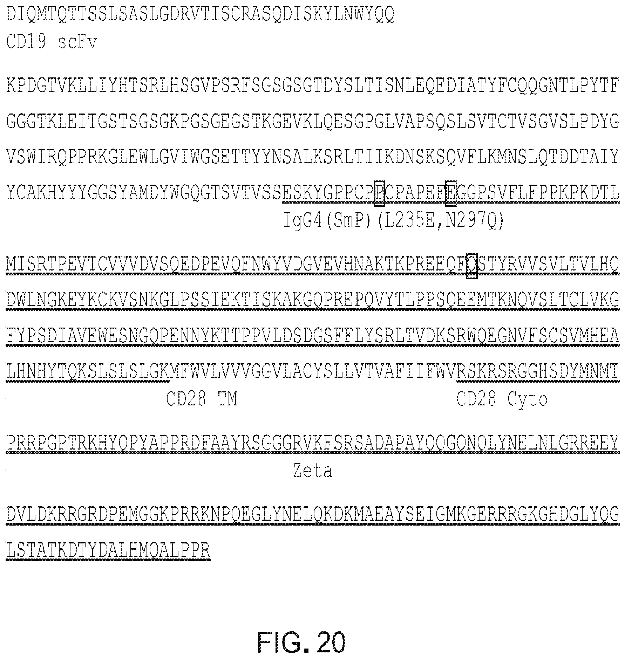

**FIG 20****: Amino acid sequence of a CD19 CAR (Mature).** SEQ ID NO:2 is the sequence of an alternate mature CD19 CAR (lacking signal sequence). The various domains are labelled and indicated by underlining or lack of underling with the exception of the GGG linker which is indicated by a double underline.

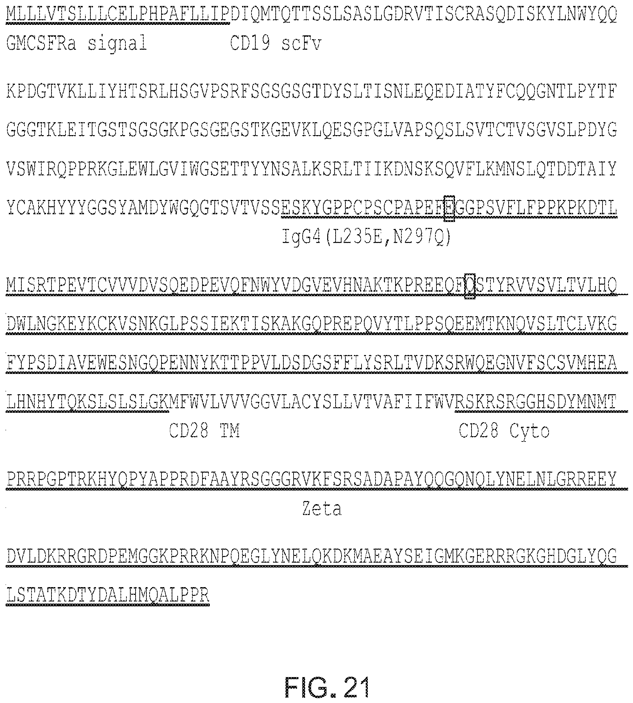

**FIG 21****: Amino acid sequence of a CD19 CAR with a Signal Sequence**. SEQ ID NO:4 is the sequence of this CD19 CAR (including the signal sequence; GMCSFRa signal peptide (SEQ ID NO: 9)) which differs from the CD19 used in the studies described herein in that the IgG4 spacer lacks the S to P mutation (spacer is SEQ ID NO: 19 rather than SEQ ID NO:20). The various domains are labelled and indicated by underlining or lack of underling with the exception of the GGG linker which is indicated by a double underline.

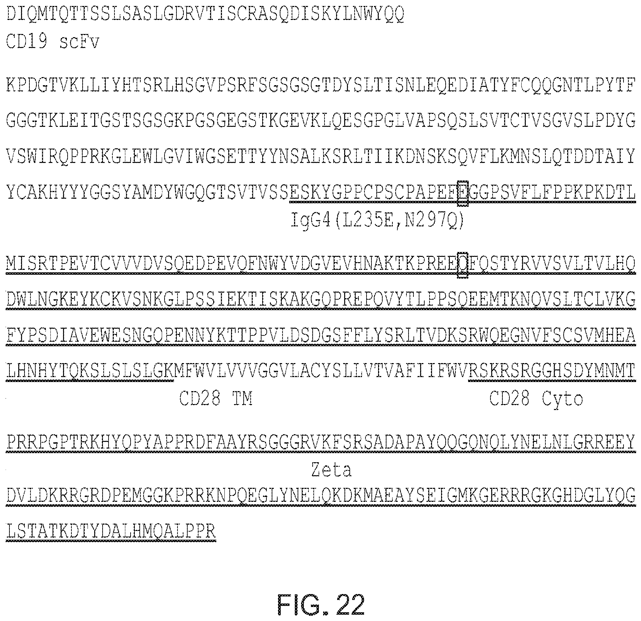

**FIG 22****: Amino acid sequence of a CD19 CAR (Mature).** SEQ ID NO:5 is the sequence of this CD19 CAR (lacking signal sequence) which differs from the CD19 CAR used in the studies described herein in that the IgG4 spacer lacks the S to P mutation (spacer is SEQ ID NO: 19 rather than SEQ ID NO:20). The various domains are labelled and indicated by underlining or lack of underling with the exception of the GGG linker which is indicated by a double underline.

### DETAILED DESCRIPTION

Described below is the structure, construction and characterization of various CD19 CAR T cells and their use in treating lymphoma of the central nervous system as well a peripheral lymphoma. A chimeric antigen (CAR) is a recombinant biomolecule that contains, at a minimum, an extracellular recognition domain, a transmembrane region, and an intracellular signaling domain. The term "antigen," therefore, is not limited to molecules that bind antibodies, but to any molecule that can bind specifically to a target. For example, a CAR can include a ligand that specifically binds a cell surface receptor. The extracellular recognition domain (also referred to as the extracellular domain or simply by the recognition element which it contains) comprises a recognition element that specifically binds to a molecule present on the cell surface of a target cell. The transmembrane region anchors the CAR in the membrane. The intracellular signaling domain comprises the signaling domain from the zeta chain of the human CD3 complex and one or more costimulatory signaling domains. CARs can both to bind antigen and transduce T cell activation, independent of MHC restriction. Thus, CARs are "universal" immunoreceptors which can treat a population of patients with antigen-positive tumors irrespective of their HLA genotype. Adoptive immunotherapy using T lymphocytes that express a tumor-specific CAR can be a powerful therapeutic strategy for the treatment of cancer.

In some cases the CAR described herein can be produced using a vector in which the CAR open reading frame is followed by a T2A ribosome skip sequence and a truncated CD19 (CD19t), which lacks the cytoplasmic signaling tail (truncated at amino acid 323). In this arrangement, co-expression of CD19t provides an inert, non-immunogenic surface marker that allows for accurate measurement of gene modified cells, and enables positive selection of gene-modified cells, as well as efficient cell tracking and/or imaging of the therapeutic T cells in vivo following adoptive transfer. Co-expression of CD19t provides a marker for immunological targeting of the transduced cells in vivo using clinically available antibodies and/or immunotoxin reagents to selectively delete the therapeutic cells, and thereby functioning as a suicide switch. In other cases, the CD19 CAR can be expressed with a truncated EGFR. The truncated EGFR provides a marker for immunological targeting of the transduced cells in vivo using clinically available antibodies and/or immunotoxin reagents to selectively delete the therapeutic cells, and thereby functioning as a suicide switch.

The disclosed compositions for use in methods of treatment using CAR T cells can be performed at various doses and across various timeframes. For example, a patient receiving an infusion, administration, or injection of CD19 CAR T cells may receive a single dose comprising between 1 × 10⁶ and 15 × 10⁶ cells. In other words, a single dose for use in the disclosed methods can comprise 1 × 10⁶, 2 × 10⁶, 3 × 10⁶, 4 × 10⁶, 5 × 10⁶, 6 × 10⁶, 7 × 10⁶, 8 × 10⁶, 9 × 10⁶, 10 × 10⁶, 11 × 10⁶, 12 × 10⁶, 13 × 10⁶, 14 × 10⁶, or 15 × 10⁶ cells. Over the entire course of treatment, a patient may receive a cumulative or total dose of cells between 20 × 10⁶ and 150 × 10⁶ T cells. For instance, the patient may receive about 20 × 10⁶, about 25 × 10⁶, about 30 × 10⁶, about 35 × 10⁶, about 40 × 10⁶, about 45 × 10⁶, about 50 × 10⁶, about 55 × 10⁶, about 60 × 10⁶, about 65 × 10⁶, about 70 × 10⁶, about 75 × 10⁶, about 80 × 10⁶, about 85 × 10⁶, about 90 × 10⁶, about 95 × 10⁶, about 100 × 10⁶, about 105 × 10⁶, about 110 × 10⁶, about 115 × 10⁶, about 120 × 10⁶, about 125 × 10⁶, about 130 × 10⁶, about 135 × 10⁶, about 140 × 10⁶, about 145 × 10⁶, or about 150 × 10⁶ or more T cells over the course of treatment. In some embodiments, a patient can receive a total dose of at least 90 × 10⁶ T cells. In one embodiment, a patient can receive a total dose of 94 × 10⁶ T cells.

Furthermore, the doses may be administered according to different regimens and timetables. For example, the disclosed methods can comprise an infusion, administration, or injection once a day, once every two days, once every three days, once every four days, once every five days, once every six days, a week, once every two weeks, once every three weeks, once every four weeks, once a month, once every other month, once every three months, or once every six months. In some embodiments, the disclosed methods can comprise continuous infusion, for instance, from a wearable pump. Similarly, the total time course of treatment may be about 5 weeks, about 10 weeks, about 15 weeks, about 20 weeks, about 25 weeks, about 30 weeks, about 35 weeks, about 40 weeks, about 45 weeks, about 50 weeks, about 55 weeks, about 60 weeks, about 65 weeks, about 70 weeks, about 75 weeks, or more. The patient may receive 1, 2, 3, 4, 5, 6, 7, 8, 9, 10, 11, 12, 13, 14, 15, 16, 17, 18, 19, or 20 or more infusions, administrations, or injections of T cells over the course of treatment according to the disclosed methods. For example, in one embodiment, a patient can receive 11 infusions of T cells over the course of 15 weeks.

Treating cancer, and more specifically lymphoma, according to the disclosed methods can result in numerous therapeutic effects. For instance, treatment with the disclosed CAR T cells can result in an increase in the level of cytokines (e.g., Th1 cytokines) and chemokines in the CSF of a patient being treated according to the disclosed methods. Cytokine and/or chemokine expression may increase by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or at least 100%, or cytokine and/or chemokine expression may increase by at least 1-fold, at least 2-fold, at least 3-fold, at least 4-fold, at least 5-fold, at least 6-fold, at least 7-fold, at least 8-fold, at least 9-fold, or at least 10-fold compared to baseline levels, as measured prior to treatment with a composition comprising CAR T cells. This increase in expression may be observed for 1, 2, 3, 4, 5, 6, 7, 8, 9, 10, 11, 12, 13, 14, or 15 or more cytokines or chemokines.

In particular, the expression of at least one of EGF, Eotaxin, FGF, G-CSF, GM-CSF, HGF, IFN-α, IFN-γ, IL-10, IL-12, IL-13, IL-15, IL-17, IL-1Rα, IL-1β, IL-2, IL-2R, IL-4, IL-5, IL-6, IL-7, IL-8, IP-10, MCP-1, MIG, MIP-1α, MIP-1β, RANTES, TNF-α, and VEGF may increase as a result of treatment with CAR T cells as disclosed herein. Furthermore, the increase in cytokine and/or chemokine expression may be local (i.e. the increase is only observable in the CNS and CFS, while serum levels of cytokines and chemokines remain unchanged.

Treatment according to the disclosed methods may also result in an increase in T cells detectable in the CSF. While at least some of the T cells detectable in the CSF following treatment will likely be CAR-expressing T cells, there may also be an increase in endogenous T cells that are recruited to the CSF. Although not wanting to be bound by theory, the increase in endogenous T cells may be a result of the recruitment of Type 1 and Type 2 T helper cells due to the increase in local cytokine levels. Additionally, the detectable T cells can comprise CD3+ T cells, as well as CD14+ CD11b+ HLA-DR+ mature myeloid populations, CD19+ B cells and CD11b+ CD15+ granulocytes, and/or reactive lymphocytes, monocytes, and macrophages.

The increase in the number of T cells in the CSF may be detectable for a specific period of time following treatment according to the disclosed methods. A detectable increase in T cells in the CSF may persist or be sustained for 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 or more days following administration of a composition comprising T cells. For example, an increase in the number of T cells observed in the CSF may not return to baseline levels (i.e. the number of T cells detectable prior to treatment) for about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days. The number of T cells detectable in the CSF may increase by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or at least 100%, or by at least 1-fold, at least 2-fold, at least 3-fold, at least 4-fold, at least 5-fold, at least 6-fold, at least 7-fold, at least 8-fold, at least 9-fold, at least 10-fold, at least 11-fold, at least 12-fold, at least 13-fold, at least 14-fold, or at least 15-fold compared to baseline levels, as measured prior to treatment with a composition comprising CAR T cells.

As used herein, the term "intraventricular" refers to the space inside the ventricular system, specifically the cerebral ventricles. Accordingly, the term "intraventricular" and "intracerebroventricular" may be used interchangeably throughout this disclosure. Accordingly, "intraventricular administration" or "intraventricular injection" refer to delivery of a composition into the ventricals of the brain (i.e. the cerebral ventricles). The cerebral ventricles are a series of interconnected, fluid-filled spaces that lie in the core of the forebrain and brainstem. This system comprises four ventricles: the right and left lateral ventricles (one of which is found in each hemisphere of the brain), the third ventricle, and the fourth ventricle.

The disclosed methods comprise various routes of administering the compositions comprising T cells. For instance, in some embodiments, the disclosed compositions may be delivered or administered intraventricularly. In some embodiments, the disclosed compositions may be delivered or administered into the spinal canal (i.e. intrathecal delivery). In some embodiments, the disclosed compositions may be delivered or administered into the epidural space of the spinal cord (i.e. epidural delivery). In some embodiments, the disclosed compositions may be delivered or administered directly into a tumor (i.e. intratumoral delivery). In some embodiments, the disclosed compositions may be delivered or administered into a cavity formed by the resection of tumor tissue (i.e., intracavity delivery). Furthermore, in some embodiments, the disclosed methods can comprise a combination of the aforementioned routes of administration. For instance, a patient may receive at least one dose of the composition comprising T cells via intracavity delivery, followed by at least one dose of the composition via intraventricular delivery.

The disclosed methods of treatment improve clinical prognosis in patients compared to current standards. For instance, the disclosed methods can increase 1-year, 2-year, and 5-year survival rates. In some embodiments, the 1-year survival rate of a patient being treated according to the disclosed methods can at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or at least 100%. In some embodiments, the 2-year survival rate of a patient being treated according to the disclosed methods can at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or at least 100%. In some embodiments, the 5-year survival rate of a patient being treated according to the disclosed methods can at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or at least 100%.

In some embodiments, the disclosed methods also increase the life expectancy of a patient compared to another patient receiving conventional treatments or SOC treatment, including radiation therapy, small molecule drug therapy, therapeutic biologics like therapeutic antibodies, or a combination thereof. In some embodiments, in which the patient receiving SOC treatment can expect to survive about 15 months from initial diagnosis (overall survival or OS), the patient receiving the disclosed treatment can expect an OS of 25, 26, 27, 28, 29, 30, 31, 32, 33, 34, 35, 36 months or more. In some embodiments, the patient receiving the claimed treatment can expect an OS of 42, 48, 54, 60, 66, 72, 78, 84, 90 months or more.

The disclosed methods may improve a patient's prognosis through a variety of clinical outcomes. For instance, the disclosed methods can result in a reduction in tumor volume in a patient being treated with a composition comprising T cells. In some embodiments, the disclosed methods of treatment can result in at least a 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or at least 100% reduction in tumor volume. In some embodiments, the tumors in a patient may be completely eliminated and the patient can be cured of the malignancy.

Additionally, the disclosed methods are safe and well-tolerated. Patients being treated according to the disclosed methods may not experience significant side effects, and furthermore, may be able to discontinue taking auxiliary medications. For instance, in some embodiments, the disclosed methods will not result in any grade 3 or higher toxicities according to NCI Common Toxicity Criteria (CTC). The CTC provides a quantifiable scale of 0-5, with 0 meaning no adverse event, 1 meaning mild, 2 meaning moderate, 3 meaning sever and undesirable, 4 meaning life threatening or disabling, and 5 meaning death. Thus, side effects and or toxicities may include events like mild or moderate headaches, fatigue, myalgia, and minor nervous system disorders such as olfactory aura, but high grade toxicities will be avoided.

The CAR described herein can be produced by any means known in the art, though preferably they are produced using recombinant DNA techniques. Nucleic acids encoding the several regions of the chimeric receptor can be prepared and assembled into a complete coding sequence by standard techniques of molecular cloning known in the art (genomic library screening, PCR, primer-assisted ligation, site-directed mutagenesis, etc.) as is convenient. The resulting coding region is preferably inserted into an expression vector and used to transform a suitable expression host cell line, preferably a T lymphocyte cell line, and most preferably an autologous T lymphocyte cell line.

Various T cell subsets isolated from the patient, including unselected PBMC or enriched CD3 T cells or enriched CD3 or memory T cell subsets, can be transduced with a vector for CAR expression. Central memory T cells are one useful T cell subset. Central memory T cell can be isolated from peripheral blood mononuclear cells (PBMC) by selecting for CD45RO+/CD62L+ cells, using, for example, the CliniMACS^{®} device to immunomagnetically select cells expressing the desired receptors. The cells enriched for central memory T cells can be activated with anti-CD3/CD28, transduced with, for example, a SIN lentiviral vector that directs the expression of the CAR as well as a truncated human CD19 (CD19t) or a truncated EGFR, a non-immunogenic surface marker for both *in vivo* detection and potential *ex vivo* selection. The activated/genetically modified central memory T cells can be expanded in vitro with IL-2/IL-15 and then cryopreserved.

In some cases, a patient suffering from a lymphoma, e.g., relapsed intermediate-grade B-cell non-Hodgkin lymphoma (NHL), will have undergone myeloablative autologous hematopoietic stem cell transplantation (HSCT) prior to treatment with CD19 CAR T cells.

### Example 1: Construction and Structure of a CD19-specific CAR

The mature amino acid sequence of the CD19-specific CAR used in the studies described below is depicted in **FIG 22** (SEQ ID NO:2). The codon optimized CAR sequence contains scFv targeted to CD19 (e.g., an scFv derived from FMC63; SEQ ID NO:3; **FIG 19B**), an IgG4 Fc spacer containing two mutations (L235E; N297Q) that greatly reduce Fc receptor-mediated recognition models (the S228P mutation is also present), a CD28(M) transmembrane domain (SEQ ID NO: 24), a costimulatory CD28(gg) (SEQ ID NO: 32) cytoplasmic signaling domain, and a CD3ζ cytoplasmic signaling domain SEQ ID NO:30). A T2A ribosome skip sequence (SEQ ID NO:7) can separates this CD19 CAR sequence from a modified EGFR (SEQ ID NO:8), a cell surface detection/selection marker lacking signaling activity. This T2A linkage results in the coordinate expression of both CD19 CAR and a modified EGFR from a single transcript. The amino acid sequence the CD19 CAR used in the studies described below is shown in **FIG 22** with the various domains indicated. In this drawing, the GMCSFRa signal sequence, the CD19 scFv, IgG4(EQ) Fc, CD28 transmembrane, CD28 cytoplasmic signaling, three-glycine linker, and CD3ζ cytoplasmic signaling domains of the CD19 CAR are indicated. **FIG 17A** depicts an amino acid sequence (SEQ ID NO:6) that can directly follow the CD3ζ amino acid sequence to permit coordinate express of the modified EGFR marker. The CD19 CAR was described and characterized previously (Wang et al. 2016 Blood 127:2980).

### Example 2: Construction and Structure of epHIV7 used for Expression of a CD19 CAR

The pHIV7 plasmid is the parent plasmid from which the clinical vector CD19-T2A-CD19t_epHIV7 was derived in the T Cell Therapeutics Research Laboratory (TCTRL) at City of Hope (COH). The epHIV7 vector used for expression of the CAR was produced from pHIV7 vector. Importantly, this vector uses the human EF1 promoter to drive expression of the CAR. Both the 5' and 3' sequences of the vector were derived from pv653RSN as previously derived from the HXBc2 provirus. The polypurine tract DNA flap sequences (cPPT) were derived from HIV-1 strain pNL4-3 from the NIH AIDS Reagent Repository. The woodchuck post-transcriptional regulatory element (WPRE) sequence was previously described.

Construction of pHIV7 is described in US 7446179. Briefly, pv653RSN, containing 653 bp from gag-pol plus 5' and 3' long-terminal repeats (LTRs) with an intervening SL3-neomycin phosphotransferase gene (Neo), was subcloned into pBluescript, as follows: In Step 1, the sequences from 5' LTR to rev-responsive element (RRE) made p5'HIV-1 51, and then the 5' LTR was modified by removing sequences upstream of the TATA box, and ligated first to a CMV enhancer and then to the SV40 origin of replication (p5'HIV-2). In Step 2, after cloning the 3' LTR into pBluescript to make p3'HIV-1, a 400-bp deletion in the 3' LTR enhancer/promoter was made to remove cis-regulatory elements in HIV U3 and form p3'HIV-2. In Step 3, fragments isolated from the p5'HIV-3 and p3'HIV-2 were ligated to make pHIV-3. In Step 4, the p3'HIV-2 was further modified by removing extra upstream HIV sequences to generate p3'HIV-3 and a 600-bp BamHI-SalI fragment containing WPRE was added to p3'HIV-3 to make the p3'HIV-4. In Step 5, the pHIV-3 RRE was reduced in size by PCR and ligated to a 5' fragment from pHIV-3 (not shown) and to the p3'HIV-4, to make pHIV-6. In Step 6, a 190-bp BglII-BamHI fragment containing the cPPT DNA flap sequence from HIV-1 pNL4-3 (55) was amplified from pNL4-3 and placed between the RRE and the WPRE sequences in pHIV6 to make pHIV-7. This parent plasmid pHIV7-GFP (GFP, green fluorescent protein) was used to package the parent vector using a four-plasmid system.

A packaging signal, psi ψ, is required for efficient packaging of viral genome into the vector. The RRE and WPRE enhance the RNA transcript transport and expression of the transgene. The flap sequence, in combination with WPRE, has been demonstrated to enhance the transduction efficiency of lentiviral vector in mammalian cells.

The helper functions, required for production of the viral vector), are divided into three separate plasmids to reduce the probability of generation of replication competent lentivirus via recombination: 1) pCgp encodes the gag/pol protein required for viral vector assembly; 2) pCMV-Rev2 encodes the Rev protein, which acts on the RRE sequence to assist in the transportation of the viral genome for efficient packaging; and 3) pCMV-G encodes the glycoprotein of the vesiculo-stomatitis virus (VSV), which is required for infectivity of the viral vector.

There is minimal DNA sequence homology between the pHIV7 encoded vector genome and the helper plasmids. The regions of homology include a packaging signal region of approximately 600 nucleotides, located in the gag/pol sequence of the pCgp helper plasmid; a CMV promoter sequence in all three helper plasmids; and a RRE sequence in the helper plasmid pCgp. It is highly improbable that replication competent recombinant virus could be generated due to the homology in these regions, as it would require multiple recombination events. Additionally, any resulting recombinants would be missing the functional LTR and tat sequences required for lentiviral replication.

The CMV promoter was replaced by the EF1α-HTLV promoter (EF1p), and the new plasmid was named epHIV7. The EF1p has 563 bp and was introduced into epHIV7 using NruI and NheI, after the CMV promoter was excised.

The lentiviral genome, excluding gag/pol and rev that are necessary for the pathogenicity of the wild-type virus and are required for productive infection of target cells, has been removed from this system. In addition, the CD19-T2ACD19t_epHIV7 vector construct does not contain an intact 3'LTR promoter, so the resulting expressed and reverse transcribed DNA proviral genome in targeted cells will have inactive LTRs. As a result of this design, no HIV-I derived sequences will be transcribed from the provirus and only the therapeutic sequences will be expressed from their respective promoters. The removal of the LTR promoter activity in the SIN vector is expected to significantly reduce the possibility of unintentional activation of host genes.

### Example 3: Production of Vectors for Transduction of Patient T Cells

For each plasmid (CD19-T2A-CD19t_epHIV7; pCgp; pCMV-G; and pCMV-Rev2), a seed bank is generated, which is used to inoculate the fermenter to produce sufficient quantities of plasmid DNA. The plasmid DNA is tested for identity, sterility and endotoxin prior to its use in producing lentiviral vector.

Briefly, cells are expanded from the 293T working cell (WCB), which has been tested to confirm sterility and the absence of viral contamination. A vial of 293T cells from the 293T WCB is thawed. Cells are grown and expanded until sufficient numbers of cells exists to plate an appropriate number of 10 layer cell factories (CFs) for vector production and cell train maintenance. A single train of cells can be used for production.

The lentiviral vector is produced in sub-batches of up to 10 CFs. Two sub-batches can be produced in the same week leading to the production of approximately 20 L of lentiviral supernatant/week. The material produced from all sub-batches is pooled during the downstream processing phase, in order to produce one lot of product. 293T cells are plated in CFs in 293T medium (DMEM with 10% FBS). Factories are placed in a 37°C incubator and horizontally leveled in order to get an even distribution of the cells on all the layers of the CF. Two days later, cells are transfected with the four lentiviral plasmids described above using the CaPO₄ method, which involves a mixture of Tris:EDTA, 2M CaCl2, 2X HBS, and the four DNA plasmids. Day 3 after transfection, the supernatant containing secreted lentiviral vectors is collected, purified and concentrated. After the supernatant is removed from the CFs, End-of-Production Cells are collected from each CF. Cells are trypsinized from each factory and collected by centrifugation. Cells are resuspended in freezing medium and cryopreserved. These cells are later used for replication-competent lentivirus (RCL) testing.

To purify and formulate vectors crude supernatant is clarified by membrane filtration to remove the cell debris. The host cell DNA and residual plasmid DNA are degraded by endonuclease digestion (Benzonase^{®}). The viral supernatant is clarified of cellular debris using a 0.45 µm filter. The clarified supernatant is collected into a pre-weighed container into which the Benzonase^{®} is added (final concentration 50 U/mL). The endonuclease digestion for residual plasmid DNA and host genomic DNA as performed at 37°C for 6 h. The initial tangential flow ultrafiltration (TFF) concentration of the endonuclease-treated supernatant is used to remove residual low molecular weight components from the crude supernatant, while concentrating the virus ~20 fold. The clarified endonuclease-treated viral supernatant is circulated through a hollow fiber cartridge with a NMWCO of 500 kD at a flow rate designed to maintain the shear rate at ~4,000 sec-1 or less, while maximizing the flux rate. Diafiltration of the nuclease-treated supernatant is initiated during the concentration process to sustain the cartridge performance. An 80% permeate replacement rate is established, using 4% lactose in PBS as the diafiltration buffer. The viral supernatant is brought to the target volume, representing a 20-fold concentration of the crude supernatant, and the diafiltration is continued for 4 additional exchange volumes, with the permeate replacement rate at 100%.

Further concentration of the viral product is accomplished by using a high speed centrifugation technique. Each sub-batch of the lentivirus is pelleted using a Sorvall RC-26 plus centrifuge at 6000 RPM (6,088 RCF) at 6°C for 16-20 h. The viral pellet from each sub-batch is then reconstituted in a 50 mL volume with 4% lactose in PBS. The reconstituted pellet in this buffer represents the final formulation for the virus preparation. The entire vector concentration process resulted in a 200-fold volume reduction, approximately. Following the completion of all of the sub-batches, the material is then placed at -80°C, while samples from each sub-batch are tested for sterility. Following confirmation of sample sterility, the sub-batches are rapidly thawed at 37°C with frequent agitation. The material is then pooled and manually aliquoted in the Class II Type A/B3 biosafety cabinet in the viral vector suite. A fill configuration of 1 mL of the concentrated lentivirus in sterile USP class 6, externally threaded O-ring cryovials is used. Center for Applied Technology Development (CATD)'s Quality Systems (QS) at COH released all materials according to the Policies and Standard Operating Procedures for the CBG and in compliance with current Good Manufacturing Practices (cGMPs).

To ensure the purity of the lentiviral vector preparation, it is tested for residual host DNA contaminants, and the transfer of residual host and plasmid DNA. Among other tests, vector identity is evaluated by RT-PCR to ensure that the correct vector is present. All release criteria are met for the vector intended for use in this study.

### Example 4: Preparation of T Cells Suitable for Use in ACT

T lymphocytes are obtained from a patient by leukopheresis, and the appropriate allogenic or autologous T cell subset, for example, Central Memory T cells (T_{CM}), are genetically altered to express the CAR, then administered back to the patient by any clinically acceptable means, to achieve anti-cancer therapy.

Suitable T_{CM} can be prepared as follows. Apheresis products obtained from consented research participants are ficolled, washed and incubated overnight. Cells are then depleted of monocyte, regulatory T cell and naive T cell populations using GMP grade anti-CD14, anti-CD25 and anti-CD45RA reagents (Miltenyi Biotec) and the CliniMACS^{™} separation device. Following depletion, negative fraction cells are enriched for CD62L+ T_{CM} cells using DREG56-biotin (COH clinical grade) and anti-biotin microbeads (Miltenyi Biotec) on the CliniMACSTM separation device.

Following enrichment, T_{CM} cells are formulated in complete X-Vivo15 plus 50 IU/mL IL-2 and 0.5 ng/mL IL-15 and transferred to a Teflon cell culture bag, where they are stimulated with Dynal ClinEx^{™} Vivo CD3/CD28 beads. Up to five days after stimulation, cells are transduced CD19 CAR-T2A-CD19t_epHIV7 lentiviral vector at a multiplicity of infection (MOI) of 1.0 to 0.3. Cultures are maintained for up to 42 days with addition of complete X-Vivo15 and IL-2 and IL-15 cytokine as required for cell expansion (keeping cell density between 3 × 10⁵ and 2 × 10⁶ viable cells/mL, and cytokine supplementation every Monday, Wednesday and Friday of culture). Cells typically expand to approximately 10⁹ cells under these conditions within 21 days. At the end of the culture period cells are harvested, washed twice and formulated in clinical grade cryopreservation medium (Cryostore CS5, BioLife Solutions).

On the day(s) of T cell infusion, the cryopreserved and released product is thawed, washed and formulated for re-infusion. The cryopreserved vials containing the released cell product are removed from liquid nitrogen storage, thawed, cooled and washed with a PBS/2% human serum albumin (HSA) Wash Buffer. After centrifugation, the supernatant is removed and the cells resuspended in a Preservative-Free Normal Saline (PFNS)/ 2% HSA infusion diluent. Samples are removed for quality control testing.

A variety of methods can be used to produce a population of human T_{CM/SCM/N} (also referred to as T_{N/MEM}) cells. For example, a population of T_{CM/SCM/N} cells can be prepared from amixed population T lymphocytes. The population of T lymphocytes can be allogenic to or autologous to the subject ultimately treated using the cells and can be obtained from a subject by leukopheresis or blood draw.

The following method is an example of one that can be used to obtain a population of T_{CM/SCM/N} cells from T lymphocytes obtained by leukapheresis or other means. Peripheral blood is collected by leukapheresis or peripheral blood draw. Day 1 of a typical manufacturing cycle is the day the ficoll procedure takes place. The subject's leukapheresis product is diluted with EDTA/PBS and the product is centrifuged at 1200 RPM for 10 minutes at room temperature with maximum brake. After centrifugation, the platelet-rich supernatant is removed and the cell pellet is gently vortexed. EDTA/PBS is used to resuspend the vortexed cell pellets in each conical tube. Each tube is then underlayed with ficoll and centrifuged at 2000 RPM for 20 minutes with no brake at room temperature. Following centrifugation, the PBMC layer from each tube is transferred into another conical tube. The cells are centrifuged at 1800 RPM for 15 minutes with maximum brake at 4°C.

After centrifugation, the cell-free supernatant is discarded and the cell pellet is gently vortexed. The cells are washed twice using EDTA/PBS each time, and a third time using PBS. Cells are centrifuged each time at 1200 RPM for 10 minutes with maximum brake at 4°C. After the final PBS wash, the vortexed cell pellet is resuspended in complete X-VIVO 15 media (X-VIVO^{™} media with 10% FBS) and transferred to a transfer bag. The bag with washed PBMC is kept overnight on a rotator at room temperature on the bench top for immunomagnetic selection the next day.

Next, selection procedures are used to both to deplete the cell population of cells expressing certain markers and to enrich the cell population for cells expressing certain other markers. These selection steps preferably occur on day two of the manufacturing cycle. The cell population is substantially depleted for cells expressing CD25 and CD14. Importantly, the cell population is not substantially depleted for cells expressing CD45RA. Briefly, cells resuspended in labeling buffer (LB; EDTA/PBS with 0.5%HSA), and incubated with anti-CD14 and anti-CD25 Miltenyi antibodies for CliniMACS^{®} depletion, and the composition is gently mixed and then incubated for 30 minutes on a rotator at room temperature on the bench top.

The depletion step is performed on a CliniMACS^{®} device using a depletion tubing set. The recovered cells following the depletion step are transferred into tubes and centrifuged at 1400 RPM for 15 minutes with maximum brake at 4°C.

The cell-free supernatant is removed and the cell pellet is gently vortexed and resuspended. To enrich for cells expressing CD62L, the cell suspension is treated with anti-CD62L-biotin (made at the City of Hope Center for Biomedicine and Genetics), gently mixed and incubated for 30 minutes on a rotator at room temperature on the bench top.

Following the incubation period, LB is added to the tube and cells are centrifuged at 1400 RPM for 15 minutes at maximum brake at 4°C. The cell-free supernatant is removed and the cell pellet is gently vortexed. LB is added to resuspend the cell pellet in the tube and the resuspended cells are transferred to a new transfer bag. Anti-biotin (Miltenyi Biotec) reagent is added and the mixture is gently mixed and incubated for 30 minutes on a rotator at room temperature on the bench top.

The CD62L enrichment step is performed on a CliniMACS^{®} device using a tubing set. The product of this enrichment can be frozen for storage and later thawed and activated To provide an intermediate holding step in the manufacturing, the option exists to freeze cells following the selection process. The cells are pelleted by centrifugation at 1400 RPM for 15 minutes with max break at 4°C. The cells are resuspended in Cryostor^{®} and aliquoted into cryovials. The vials are transferred to a controlled cooling device that can cool at about 1°C /minute (e.g., a Nalgene^{®} Mr. Frosty; Sigma-Aldrich) the cooling device is immediately transferred to a -80°C freezer. After three days in the -80°C freezer, the cells are transferred into a GMP LN2 freezer for storage.

We have found that cryopreserved cells exhibit good recovery and viability, maintain the appropriate cell surface phenotype when thawed several months after cryopreservation, and can be successfully transduced and expanded in vitro upon thawing.

Alternatively, freshly enriched Tcm/scm/n cells can be activated, transduced or expanded as described in Example 3, below.

### Example 5: Lentiviral Transduction of T_{CM/SCM/N} cells

A T_{CM/SCM/N} cell population can be transduced with a lentivirus capable of expressing a protein or proteins of interests. The following is an example of one method that can be used to transduce the T cells with a lentivirus vector with protamine sulfate/cytokine solution.

If the cells are frozen subsequent to the selection process, the cells will need to be thawed upon continuation of the manufacturing process. The cryopreserved vials are thawed in a 37°C water bath until mostly liquid remains. The contents of the vials are placed in a conical tube containing cold X-Vivo 15 media. The cells are centrifuged at 1200 RPM for 10 minutes with max brake at 4°C. After centrifugation, the cell-free supernatant is decanted, the pellets are vortexed, and all the pellets combined into one tube in X-Vivo 15 media to be transduced with lentivirus. The cell numbers are determined and the manufacturing process continues with Dynal bead activation.

The cells are centrifuged at 1400 RPM for 10 minutes at 25°C. After centrifugation, the cell-free supernatant is removed and the cell pellet is gently vortexed and resuspended in complete X Vivo 15 media to be transduced with lentivirus. If thawed cells are being used, the centrifugation step is not necessary.

Tcm/scm/n cells are stimulated with Dynabeads^{®} Human T expander CD3/CD28 (Invitrogen) at a 1:3 ratio (T cell:bead), then cultured in the presence of 50 U/mL rhIL-2 (Chiron) and 0.5 ng/ml rhIL-15 (CellGenix).

After incubation for 1-3 days, T cells are transduced with the lentiviral vector at a MOI typically ranging from 0.1 to 1.5 in X Vivo15 containing 10% FCS with 5µg/mL protamine sulfate (APP Pharmaceutical), 50 U/mL rhIL-2 and 0.5 ng/mL rhIL-15. The day following the lentivirus transduction, one volume of media is added of freshly prepared media/cytokine mastermix containing complete X-VIVO 15 media and cytokines (10 µL rhuIL-2 and 0.5 µL rhuIL-15).

Cultures are then maintained at 37°C, 5% CO₂ in X-Vivo15 10% FCS as required to keep cell density between 3×10⁵ and 2×10⁶ viable cells/mL, with cytokine supplementation (final concentration of 50 U/mL rhIL-2 and 0.5 ng/mL rhIL-15) every Monday, Wednesday and Friday of culture.

CD3/CD28 Dynabead bead removal takes between days 7 to 9 after bead stimulation. The Dynal beads are removed using the DynaMag-50 or MPC magnet. After bead removal, a sample of the cell suspension is taken for counts. Based on the counts, the concentration is adjusted to approximately 0.5×10⁶/mL.

Cultures are propagated until sufficient numbers of cells are achieved, which is typically between 8 and 18 days. During this time, a full replacement of cytokines are added to the T cell cultures. Starting on day 12 and every Monday, Wednesday and Friday thereafter, counts are taken to maintain a concentration of approximately 0.5×10⁶ cells/mL

Final harvest and cryopreservation proceeds as follows. The cell product is cryopreserved once the expanded cells reach the necessary number of viable cells required for clinical administration or research purposes. The cells are counted and in-culture samples are harvested for mycoplasma testing. The remaining cells are centrifuged at 1800 RPM for 20 minutes, maximum brake at 4°C. The cell-free supernatant is removed and all cell pellets are gently vortexed, resuspended and combined into Isolyte buffer (Braun). The tube is centrifuged at 1800 RPM for 20 minutes, max brake at 4°C. The cells are resuspended in Isolyte for a second wash.

After the second Isolyte wash, the cell-free supernatant is removed and the cell pellet is gently vortexed and resuspended in an appropriate volume of the cryopreservation media, CryoStor^{®} 5 (BioLife Solutions), in order to have cells at an appropriate concentration. Cells are cyropresserved by placing in a controlled cooling device that can cool at about 1°C /minute (e.g., a Nalgene^{®} Mr. Frosty; Sigma-Aldrich) or a control rate freezer system (Custom Biogenics). At completion of the freeze procedure, the cassette(s)/bag(s) and cryovials are immediately transferred into a LN2 freezer for storage.

### Example 6: Phenotype of T_{CM} and T_{N/MEM}-derived CD19CAR T cells

T_{CM} (CD62L+CD45RO+) and T_{N/MEM} (CD62L+) cells were isolated from healthy donor PBMCs and transduced to express a second generation CD19 specific chimeric antigen receptor (CAR) with a truncated EGFR (EGFRt). CAR T cells were expanded in the presence of IL-2 50 U/mL and IL-15 0.5ng/mL for 14 days. CAR expression was defined by Erbitux-biotin and streptavidin (SA) staining as shown in **FIG 2****.** Both T_{CM} and T_{N/MEM} T cell populations express comparable levels of CD19 CAR as indicated by EGFR staining and CD8 T cells.

### Example 7: T_{N/MEM}-derived CD19CAR T cells exhibit superior anti-CNSL activity to T_{CM}-derived CD19CAR T cells

NSG mice were injected intracranially (IC) with 0.2×10⁶GFP+ ffluc+ Daudi cells (**FIG 3A**). Five days later, mice received an IC local infusion of 2×10⁶ untransduced mock T cells, T_{CM} or T_{N/MEM}-derived CD19 CAR T cells and tumor signals were monitored by bioluminescent imaging once a week (**FIG 3B**). Kaplan-Meier survival curves (**FIG 3C****;** N=5) show that T_{N/MEM} CD19 CAR are superior to T_{CM} CD19 CAR.

### Example 8: Comparing efficacy of T_{N/MEM}-derived CD19CAR T cells against CNSL via three different delivery routes

As shown schematically in **FIG 4**, NSG mice were injected intracranially (IC) with 0.2×10⁶ GFP+ ffluc+ Daudi cells. Tumor was allowed to engraft for five days before mice received T_{N/MEM}-derived CD19 CAR T cells via three different delivery routes: 1) intracranial (IC) local infusion with 1.2×10⁶ CD19 CAR T cells, 2) intracerebroventricular (ICV) administration with 1.2×10⁶ CD19 CAR, and 3) intravenous (IV) injection with 3×10⁶ CD19 CAR T cells. Tumor growth was evaluated by bioluminescent imaging once a week.

Both IC and ICV delivery of CD19 CAR T cells were able to completely eradicate CNS lymphoma in all mice by day 14 post CAR T cell infusion. IV infusion induced significant delayed antitumor activity 21 days post T cell infusion (**FIG 5**) as compared with ICV delivered CD19CAR T cells.

### Example 9: CD19 CAR T cells are detected in the peripheral blood of IV and ICV treated mice

Twenty-eight days after CAR T cell infusion, peripheral blood was collected retro-orbitally. Percentages of CAR T cells (CD45+ Erbitux+) are depicted in **FIG 6****.**

### Example 10: Comparison of T_{N/MEM}-derived CD19 CAR T cells administered IV and ICV

NSG mice were injected subcutaneously (SC) with 3×10⁶ GFP+ ffluc+ Daudi cells and 14 days later intracranially (IC) with 0.2×10⁶ Daudi cells. Five days following IC tumor inoculation, 2×10⁶ untransduced mock T cells or T_{N/MEM}-derived CD19 CAR T cells were administered either ICV or IV. This protocol is shown schematically in **FIG 7**. Both brain and flank tumors show faster regression in mice treated with CD19 CAR T cells administered ICV as compared to those treated with IV delivered CD19 CAR T cells (**FIG 8**).

### Example 11: T_{N/MEM}-derived CD19 CAR T cells administered ICV are more efficacious than IV delivered CAR T cells in controlling both CNSL and systemic lymphoma

CD19+fflu+ tumor burden was monitored weekly. Flux (photons/sec) was determined by measuring bioluminescence with imaging once a week. CD19 CAR T cells administered ICV demonstrate the greatest ability to target both systemic (**FIG 9A**) and CNS lymphoma (**FIG 9B**).

### Example 12: ICV administered T_{N/MEM}-derived CD19CAR T cells exhibited greater proliferative capacity than IV delivered ones in lymphoma mice

CD19+fflu+ tumor was grafted on the right flank of the mice. Nineteen days after tumor engraftment, CAR T cells that were transduced with GFPffluc were administered via ICV or IV. CAR T cell proliferation was determined by measuring bioluminescence with imaging every other day. The results are depicted in **FIG 10**.

### Example 13: CD19 CAR T cells administered ICV persist in vivo and maintain antitumor activity against tumor re-challenge

Daudi lymphoma cells were engineered to express firefly luciferase and injected into mouse brain as CNS lymphoma model. After tumor engraftment, mice received 2×10⁶ CD19 CAR T cells administered intracerebroventricularly. One hundred days post CAR T cell treatment, blood was collected from the mice and human T cells and CAR T cells were analyzed with flow cytometry (**FIG 11A**). These mice were re-challenged by injecting new Daudi tumor cells on the right flank of mice. Tumor growth was monitored by measuring bioluminescence with imaging (**FIG 11B**).

### Example 14: Intracerebroventricularly administered CAR T cells completely eradicate not only CNS lymphoma, but also systemic lymphoma

As described above, CNS lymphoma and systemic lymphoma was established in mouse model by inoculating Daudi cells in the brain and SC in the right flank into the same mouse. Fifteen days post CAR T cell infusion, both CNS lymphoma and systemic tumor disappeared in the ICV treated mice (**FIG 8**, **FIG 9** and **FIG 12A**) and all mice survived tumor-free for over 300 days until the experiment was terminated (**FIG 12E**). However, the same number of CAR T cells delivered through IV showed a dramatically delayed response. Complete remission was observed 43 days post CAR T cell infusion, but tumors eventually relapsed. All IV treated mice died before day 200 and imaging data indicate that mice died of both CNS lymphoma and systemic lymphoma relapse (**FIG 12A**), suggesting that IV CAR T cells failed to completely eradicate either CNS or systemic lymphoma, which could be the result of impaired CAR T cell function and persistence.

### Example 15: Intracerebroventricularly administered CAR T cells retain memory phenotype and function with a broad TCR vbeta repertoire and ability to proliferate in vitro

Blood samples were collected at the end points (334 days for ICV and up to 180 days for IV) and analyzed by flow cytometry. CAR T cells were detected in both ICV and IV treated groups. Interestingly, even though no tumors were detected in the ICV treated mice, human CAR T cells were detected in the blood and the majority of CAR T cells were CD4 positive (**FIG 12 C-D**), indicating that ICV-infused CAR T cells have the capability of homeostatic proliferation and the persisting CD4 CAR T cells, which express significantly higher levels of CD28 (p=0.01), contribute to curing disease. To understand whether the superior antitumor activity is due to clonal or polyclonal expansion, human T cells harvested from mouse spleen 334 day after ICV treatment and 180 days post IV treatment were re-stimulated with REM method containing OKT3, irradiated PBMC, and lymphoblastoid cell lines (LCL) for 14 days and their TCR repertoire was analyzed. The data indicate that there is no skewed TCR Vbeta usage of the ICV-delivered CAR T cells, therefore it is a generalizable feature of CAR T cells **(****FIG 12E****),** similar to input cell product before infusion.

To further investigate the stem-like feature of CSF nurtured CAR T cells, an *in vitro* serial re-stimulation with the T cells harvested from mice (334 days of ICV mice and 180 days of IV mice) was performed. T cells were stimulated with OKT3 and feeders, including PBMC and LCL, for 2 cycles (14 days/cycles). CAR T cells from 2 of 2 ICV treated group expanded from 8% after REM1 to 79% after REM2, whereas CAR T cells from IV treated mice were less expanded or decreased (**FIG 13A-C**), suggesting ICV CAR T cells, but not IV CAR T cells, are able to continue to expand with repetitive in vitro stimulation. In spite of 334 days of *in vivo* incubation and repeated *in vitro* stimulation, the CAR T cells from ICV treated mice maintained higher levels of CD127, CD62L, and CD161-markers that represent central memory T cells and memory stem cells with high efflux capacity when compared with CAR T cells from IV treated mice (**FIG 13D-E**). Furthermore, functional studies of the T cells demonstrated that the CD62L positive cells exhibited significantly lower levels (p=0.01) of uptake of a glucose analog, NBDG, than CD62L negative cells (**FIG 14A-C**), supporting the memory function and phenotype of the cells. To meet different bioenergetic requirements, effector T cell development involves activation of mitochondrial fission and subsequent fragmentation, while memory T cell development involves inhibition of mitochondrial fission, leading to mitochondrial elongation. Here T cells from CAR-treated mice were harvested, *in vitro* expanded and analyzed for mitochondrial morphology. Data shows that T cells from ICV and IV treated mice possess distinct mitochondrial morphologies. ICV treated mice maintain fused networks and appear elongated, while T cells form IV treated mice have punctate mitochondria (**FIG 14D**). This observation is in line with the memory phenotype and function of T cells seen post ICV delivery, suggesting that mitochondrial remodeling is a signaling mechanism that instructs T cell metabolic programming, which is acquired during CSF conditioning.

### Example 16: Intracerebroventricularly administered CAR T cells exhibit efficient trafficking and superior proliferative and persistence potential when compared with IV delivered CAR T cells

To understand and compare the differences in trafficking and expansion following ICV and IV CAR delivery, CD19-CAR T cells were transduced with GFPffluc (**FIG 15A**) and administrated ICV and IV into NSG mice bearing SC Daudi tumors in the right flank. ICV delivered CAR T cells were able to migrate to the SC tumors as quickly and efficiently as IV delivered CAR T cells. Supportively, the CAR T cells infiltrated SC tumors and were double positive for CD3 and CAR by IHC staining. Interestingly CAR T cell signals were significantly higher in ICV mice than IV mice (P<0.001) (**FIG 15A-B**) and CAR T cells persist in ICV mice while CAR T cells in IV mice decline dramatically from 70% on day 13 to 13% on day 28 (P<0.05) (**FIG 15C-D**). This expansion is antigen dependent as mock T cells did not expand *in vivo.* Consistently, in a separate short-term experiment, ICV CAR T cells exhibited more tremendous expansion (P<0.01) and superior antitumor activity than IV CAR treated mice.

### Example 17: CSF microenvironment reprograms CAR T cell for better memory and less exhaustion

The results described above raise the possibility that exposure of CAR T cells to the CSF via ICV infusion may reprogram CAR T cells to execute potent anti-CNS lymphoma, and subsequently, anti-systemic lymphoma activity once they migrate to peripheral circulation, as well as to maintain T cell memory.

Compared with blood, CSF is cell-free and protein-free. In addition, there are other elements of difference between CSF and blood such as the lower levels of glucose and potassium in the CSF compared with serum. Glucose and potassium are known to play a key role in T cell memory and function.

To confirm that levels of glucose and potassium and other factors in CSF play a role in CAR T cell phenotype and to understand the characteristics of CSF and RPMI treated CAR T cells, RPMI 1640 (Invitrogen) was modified to contain 60mg/dL glucose 2.8 mEq/L potassium, the same concentrations of glucose and potassium as in CSF, in comparison to regular RPMI containing 200 mg/dL glucose and 5.3 mEq/L potassium. CAR T cells from 4 different donors were transduced and expanded in the modified RPMI (13 days). These cells expressed elevated levels of CD28, which is known to be associated with better expansion and persistence upon adoptive transfer, than cells cultured in regular RPMI medium (N=4, P<0.01). This change was mainly reflected in the CD4 subset (FIG 16A), which is consistent with the dominant CD4 CAR persistence in the in vivo experiment. No differences in exhaustion were observed between the two culture conditions (**FIG 16A**). Based on these data, CD19-CAR T cells were cultured in RPMI and human CSF for 48 hours in the presence of target Daudi cells. As shown in FIG 16B, CD19-CAR T cells in CSF tend to express comparable levels of exhaustion markers as those in RPMI, but higher levels of memory markers, such as IL-7alpha. These observations were consistent among cells collected from four different healthy donors, and indicate that metabolic conditions may contribute to better CAR T cell function through T cell lineage polarization and improved CAR T cell memory of ICV infused CAR T cells.

Next, CD19-CAR T cells were cultured in in RPMI and CSF for 48 hours and an RT-PCR array of genes associated with memory, effector function, and metabolism was run. Across 4 different donors, elevated expression (>1.3 fold change) of genes related to better survival and memory function, such as BCL2, BCL2L11 KLF2, and IL7Ralpha(CD127) was observed and decreased expression of terminal differentiation genes like PRF1, IFNgamma, GZMB, and TBX21 (>1.3 fold change) was observed in the cells treated with CSF when compared with RPMI treated CAR T cells (**FIG 16C**). In spite of upregulated HK2, which may be due to compensation for the low glucose condition, glycolytic flux enhancer, Pgam-1, that impairs the memory T cell formation, was downregulated in CSF cultures. In addition, numerous other genes regulating glucose metabolism such as CPT1A, an enzyme that controls the rate-limiting step for fatty oxidation (FAO) and glycolysis was decreased. Along with down regulated LDHA, the metabolic pathway of CAR T cells in CSF may switch to mitochondrial oxidative phosphorylation as the main source of ATP, favoring the establishment of T cell memory that is supported by elongated mitochondria in ICV CAR T cells as shown in **FIG 14**.

Furthermore, CAR T cells treated in CSF tend to produced significantly lower levels of effector cell related and T cell inhibitory cytokines upon stimulation with Daudi cells (**FIG 16D**), a finding consistent with the transcriptional profiles of less differentiated cells treated with CSF (**FIG 16C**). When CAR T cells were stimulated with microbeads coated with soluble CD19 protein, which is recognized by CD19 CAR scFv (FMC63), and used as stimulator for CAR T cells at a ratio of 1:1 in RPMI and CSF for 48 hours, cells in CSF expressed upregulated activation-related genes, such as PRDM1, PDCD1, and FOXP3, but simultaneously also upregulated KLRB1 (CD161), BCL2 and down regulated B3GAT1 (CD57) ppp2r2, GZMB, EOMES, and again, the low glycolytic gene, CPT1A. Overall, the data here suggest that conditions in the CSF can reprogram CD19-CAR T cells through upregulation of memory genes and down regulation of glycolysis-associated genes, resulting in a unique CAR T cell population that is fully activated for immediate antitumor function as well as acquired memory function.

### Example 18: Antigen-primed CAR T cells possess memory phenotype and are able to resist tumor re-challenge

As shown above, ICV CAR T cells not only efficiently eradicate brain tumor, but also systemic lymphoma. To elucidate whether this is the result of CSF reprograming alone or a combination effect with T cell activation by the brain tumor before they migrate to the peripheral blood, an in vitro priming model was developed by co-culturing CAR T cells with irradiated tumor cells for 1 hour before injecting via ICV into mice bearing s.c. tumor in right flanks. Flank tumors showed faster regression in mice treated with in vitro primed CD19-CAR T cells as compared to those treated with un-primed CD19-CAR T cells (P=0.003) (**FIG 17A** **and** **FIG 17D**). 133 days post CAR infusion, the surviving mice were re-challenged with new tumor. 100% of primed and 60% un-primed CAR T treated mice remained tumor free for more than 200 days following re-challenge (**FIG 17C-D**), indicating that CSF alone is able to improve antitumor activity and antigen priming would further enhance antitumor activity and T cell memory.

Because the enhanced CAR T cell function after antigen priming could be independent of CSF, CD19-CAR T cells were primed and administered them intravenously. Mice with systemic tumors (s.c.) were treated with primed and un-primed CD19-CAR T cells. Consistent with previous observations, primed CAR T cells induced better tumor regression when compared with unprimed CAR T cells (P<0.01) **(****FIG 17E-F**). All mice that received un-primed CD19-CAR T cells died before or around day 90. In contrast, three of the five mice that received primed CAR T cells survived longer than 135 days and two of these three mice resisted tumor re-challenge (**FIG 17G**). Regardless of significantly prolonged survival (p<0.01) when compared with un-primed CAR T cell-treated mice, mice receiving primed CAR T cells start to decline around 200 days (**FIG 17H**), which is inferior survival when compared to their ICV counter parts. Along with the improved efficacy of primed CAR T cells, 133 days after CAR T cell infusion, there are significantly higher levels of CAR T cells persisting in the primed ICV treated mice than that of unprimed ICV and primed IV CAR treated mice (**FIG 18A-B**). This data support the hypothesis that both CSF reprograming and T cell activation by the brain tumor synergistically augment CAR T cell antitumor efficacy.

### Methods and Materials

***Antibodies and Flow Cytometry*** Fluorochrome-conjugated isotype controls against CD3, CD4, CD8, PD1, KLRG, CD28, CD62L, CD127, and streptavidin-PE were obtained from BD Biosciences. The antibody against EGFR was purchased from BioLegend. Biotinylated Erbitux was generated from cetuximab purchased from the City of Hope pharmacy. The IOTestBeta Mark TCR Vβ Repertoire Kits (representing ~70% of normal TCR Vβ repertoire) were obtained from Beckman Coulter. Isotype-matched mAbs served as controls. Data acquisition was performed on a MACSquant (Miltenyi) and analyzed using FCS Express Version 3 software (De Novo Software). 2-NBDG was obtained from Fisher Scientific.

***Lentivirus Vector Construction and CAR T cell generation*** The lentivirus CAR construct was modified from the previously described CD19-specific scFvFc:ζ chimeric immunoreceptor (Kowolik et al. 2006 Cancer Research 66:10995). The CD19R:CD28:ζ/EGFRt-epHIV7 contains: 1) the CAR sequence consisting of the V_{H} and V_{L} gene segments of the CD19-specific FMC63 mAb, an IgG4 hinge with mutations at two sites (L235E; N297Q) within the CH₂ region, the transmembrane and cytoplasmic domains of the costimulatory molecule, CD28, and the cytoplasmic domain of the CD3ζ chain; 2) the ribosomal skip T2A sequence; and 3) the truncated human EGFR (EGFRt) sequence. Leukapheresis products were obtained from healthy donors using protocols approved by the City of Hope Institutional Review Board. Naive and central memory T cells defined as CD62L+CD14- were isolated on a Miltenyi CliniMACS (Miltenyi Biotec, Inc.). Briefly, Leukapheresis products were obtained from consented healthy donors, PBMC were isolated by density gradient centrifugation over Ficoll-Paque (GE Healthcare), and incubated with anti-CD14 microbeads (Miltenyi Biotec, Inc.) for 30 minutes at RT. CD14+ cells were then immediately depleted using the CliniMACS^{™}. The unlabeled negative fraction of cells was labeled with biotinylated-DREG56 mAb (COH CBG) followed by incubation with anti-biotin microbeads (Miltenyi Biotec, Inc.). The CD62L⁺ cells were purified with positive selection on the CliniMACS^{™}. Right after selection, the T cells were activated, transduced, and maintained as described (Wang 2012 J Immunotherapy 35:689). In some experiments, CAR T cells were transduced to express ffluc and GFP.

***Reverse transcriptase (RT) quantitative PCR (qPCR) analysis*** RNA was extracted with RNeasy Kits (Qiagen). RT-qPCR was performed using the RT² Profiler PCR Array (SABiosciences) designed with customized primers for sequences of transcriptionally regulated genes shown to impart differentiation, survival, metabolism, and apoptotic attributes to CAR T cells (Hinrichs CS, Blood 2011; Willinger TJ Immunol 2005). Four control genes, including housekeeping genes PPIA (Peptidylprolyl isomerase A; cyclophilin A), RPL13A (Ribosomal protein L13a), control for reverse transcription, a positive PCR control were also used. All samples passed RNA quality control, PCR array reproducibility, RT efficiency, and genomic DNA contamination tests performed according to the manufacturer's instructions. Data were analyzed by ΔΔCₜ method using RT² Profiler PCR Array Web portal software (http://www.sabiosciences.com). Median fold change of CSF versus RPMI cultured CAR T cells from 4 individual donors are presented. Hierarchical clustering analysis was performed in Cluster 3.0 using the average linkage clustering method, with genes demonstrating an absolute fold change larger than 1.3. The results were then visualized in Treeview 1.1.6r4 and a heatmap was generated.

***Cytokine production assays*** Expanded CAR T cell products (10⁵) were co-cultured in 96-well tissue culture plates with 10⁵ CD19+ stimulator cells (Daudi). Supernatants were harvested 18 h after stimulation and analyzed in triplicates by cytometric bead array using the Bio-Plex Human Cytokine 17-Plex Panel (Bio-Rad Laboratories) according to the manufacturer's instructions.

***NBDG uptake assays*** 2-NBDG is a fluorescent glucose analog that has been used to monitor glucose uptake in live cells. NBDG fluorescence typically displays excitation/emission maxima of ~465/540 nm and can be visualized using a FACS machine with a green/FITC channel. Cells were washed with warm PBS twice and stained with an APC-conjugated anti human CD62L antibody for 15 minutes at RT. After staining, the cells were washed two times with warm PBS. The cells were then cultured in 100 µM 2-NBDG PBS buffer for 10 minutes and 30 minutes in a 5% CO₂ incubator at 37°C. After two washes with PBS, the cells were analyzed with the MACquant.

***Immunohistochemistry staining (IHC)*** Fresh tissue was collected from mice with flank (s.c.) tumors, fast frozen, and embedded in O.C.T. The tumor tissue was cut into 10 µM thick sections and placed on slides. To detect CAR T cells within the tumor, the slides were double stained with primary antibodies for CD3 (LS-B8669, rabbit anti-human) and EGFR (280005, mouse anti-human). Briefly, air dried and fixed slides were blocked by 1% donkey serum in PBS buffer and incubated with primary antibodies against CD3 (1:200 dilution) and EGFR (1: 100 dilution) at 4°C overnight. Following staining with 488 Alexa Flour donkey antimouse and 555 Alexa Flour donkey anti-rabbit antibodies (Invitrogen) for 30 minutes in dark, the slides were stained with 0.1% DAPI for 3 minutes and covered with ProLong Gold Antifade Mountant (Invitrogen). The cell images were processed under a Zeiss Observer II microscope.

Spleens were also collected from tumor-bearing mice following CAR T cell treatment. Human T cells harvested from the mouse spleens were re-stimulated with REM method containing OKT3, irradiated PBMC, and lymphoblastoid cell lines (LCL) as previously described (Crossland KD, J Immunol 1991). The stimulated cells were stained in T cell medium with 100 nM MitoTracker (Green) Mitochondrial selective probe for 30 minutes at 37°C. The cells were then washed 3 times with warm X-vivo medium followed by washing with PBS containing 1% FBS. 1×10⁵ cells were plated onto slides using a cytospin (700 RPM/h, 3 minutes) and the slides were dried overnight. The following day, cells were fixed with 4% PFA for 20 minutes and stained with DAPI for 1 minute. The mitochondrial images were taken using a LSM880 confocal microscope.

***Orthotopic brain and systemic lymphoma xenograft model*** Six- to ten-week old NOD/*Scid* IL2RγC^{null} mice were injected intracranially and/or subcutaneously with 2 × 10⁶ fflucGFP Daudi cells on day 0. Five days after intracranial injection and 19 days post subcutaneous injection, mice were treated with CD19 CAR T cells (i.v. or i.c.v.) or non-transduced mock cells. Anesthetized mice were imaged using a Xenogen IVIS 100 series system (Xenogen). Photons from ffLuc⁺ tumor xenografts were quantified using the software program, Living Image (Xenogen), and the bioluminescence signal was measured as total photon flux normalized for exposure time and surface area and expressed in units of photons per second per cm² per steradian. Human T cell engraftment in peripheral blood, bone marrow, and spleen was determined by flow cytometry after staining with antibodies against human CD45, CD8, and CD3, and cetuximab for CAR detection.

***Statistical Analysis*** Analyses were performed using Prism (GraphPad Software, Inc.). Log-rank (Mantel-Cox) test, Mann-Whitney test, and Kruskal-Wallis test were used to ascertain statistical significance of the in vivo data. The paired t-test (2-tailed) and two-way ANOVA were used for the statistical analysis of in vitro data.

## Claims

1. A composition comprising T cells expressing a CD19 targeted CAR for use in a method of treating central nervous system lymphoma, systemic lymphoma with concurrent central nervous system involvement, central nervous system B cell leukemia or systemic B cell leukemia with concurrent central nervous system involvement, the method comprising introducing into the cerebrospinal fluid (CSF) of a patient said composition comprising an effective amount of said T cells.

2. The composition for use of claims 1, wherein the T cells are autologous or allogenic T cells.

3. The composition for use of claim 1, wherein the composition is administered intraventricularly, or wherein the composition is administered to the central canal of the spinal cord.

4. The composition for use of claim 1, wherein the composition comprises at least 1 × 10⁶ cells, or wherein a composition comprising T cells is administered at least two times.

5. The composition for use of claim 1, wherein the lymphoma is non-Hodgkin lymphoma.

6. The composition for use of claim 1, wherein the CD19 targeted CAR comprises an amino acid sequence selected from the group consisting of SEQ ID NOs: 10, 1, 2, 4 and 5.

7. The composition for use of claim 1, wherein the method is performed after myeloablative autologous hematopoietic stem cell transplantation.

8. The composition for use of claim 1, wherein the T cells comprise both CD4+ cells and CD8+ cells, or wherein the T cells have undergone *ex vivo* expansion.

9. The composition for use of claim 1, wherein the T cells comprise at least 10%, 20%, 30%, 40%, 50% or 60% central memory T cells (T_{CM} cells), or wherein the T cells comprise at least 10%, 20%, 30%, 40%, 50% or 60% naive T cells/memory T cells (T_{N}/_{MEM} cells).

10. The composition for use of claim 1, wherein an increased level of T cells is detectable in the CNS of the patient after treatment.

11. The composition for use of claim 10, wherein the T cells detectable in the CSF comprise endogenous Type 1 T cells, or wherein the T cells detectable in the CSF comprise endogenous Type 2 T cells, or wherein the T cells detectable in the CSF comprise CD3+ T cells, or wherein the T cells detectable in the CSF comprise CD14+ CD11b+ HLA-DR+ mature myeloid populations, or wherein CD19+ B cells and CD11b+ CD15+ granulocytes are detectable in the CSF following administration of the composition, or wherein reactive lymphocytes, monocytes, and macrophages are detectable in the CSF following administration of the composition.

12. The composition for use of claim 1, wherein the CD19 CAR comprising a scFv that binds to CD19, a spacer domain, a transmembrane domain, at least one costimulatory domain and a CD3ζ signaling domain.

13. The composition for use of claim 12, wherein the scFv comprises SEQ ID NO:3, and/or wherein the spacer domain comprises any of SEQ ID NOs:11-22, and/or wherein the transmembrane domain comprises any of SEQ ID NOs: 22-29, and/or wherein the c0-stimulatoy domain comprises any of SEQ ID NOs: 31-34, and/or wherein the CD3ζ signaling domain comprises SEQ ID NO: 30.

14. The composition for use of any of claims 1-5 or 7-12, wherein the CD19 CAR comprises the amino acid sequence of SEQ ID NO: 10 and an amino acid sequence of an ScFv that binds human CD19.

15. The composition for use of any of claims 1-14, wherein the T cells expressing a CD19 targeted CAR are T cells that have been cultured in a culture medium having 60mg/dL glucose and 2.8 mEq/L potassium, or wherein the T cells expressing a CD19 targeted CAR are T cells that have been activated by exposure to CD19 prior to administration, or wherein the T cells expressing a CD19 targeted CAR are T cells that have been activated by exposure to cells expressing CD19 prior to administration.

## Patentansprüche

1. Zusammensetzung, umfassend T-Zellen, die einen CD19-zielgerichteten CAR exprimieren, zur Verwendung bei einem Verfahren zur Behandlung von Lymphom des Zentralnervensystems, systemischem Lymphom mit gleichzeitiger Beteiligung des Zentralnervensystems, Zentralnervensystem-B-Zell-Leukämie oder systemischer B-Zell-Leukämie mit gleichzeitiger Beteiligung des Zentralnervensystems, wobei das Verfahren Einführen der eine wirksame Menge der T-Zellen umfassenden Zusammensetzung in den Liquor (Cerebrospinal Fluid, CSF) eines Patienten umfasst.

2. Zusammensetzung zur Verwendung nach Anspruch 1, wobei es sich bei den T-Zellen um autologe oder allogene T-Zellen handelt.

3. Zusammensetzung zur Verwendung nach Anspruch 1, wobei die Zusammensetzung intraventrikulär verabreicht wird oder wobei die Zusammensetzung an den zentralen Rückenmarkskanal verabreicht wird.

4. Zusammensetzung zur Verwendung nach Anspruch 1, wobei die Zusammensetzung wenigstens 1 × 10⁶ Zellen umfasst oder wobei eine T-Zellen umfassende Zusammensetzung wenigstens zweimal verabreicht wird.

5. Zusammensetzung zur Verwendung nach Anspruch 1, wobei es sich bei dem Lymphom um Non-Hodgkin-Lymphom handelt.

6. Zusammensetzung zur Verwendung nach Anspruch 1, wobei der CD19-zielgerichtete CAR eine Aminosäuresequenz ausgewählt aus der Gruppe bestehend aus SEQ ID NO: 10, 1, 2, 4 und 5 umfasst.

7. Zusammensetzung zur Verwendung nach Anspruch 1, wobei das Verfahren nach myeloablativer Transplantation autologer hämatopoetischer Stammzellen durchgeführt wird.

8. Zusammensetzung zur Verwendung nach Anspruch 1, wobei die T-Zellen sowohl CD4+-Zellen als auch CD8+-Zellen umfassen oder wobei bei den T-Zellen eine Ex-vivo-Expansion erfolgte.

9. Zusammensetzung zur Verwendung nach Anspruch 1, wobei die T-Zellen wenigstens 10%, 20%, 30%, 40%, 50% oder 60% zentrale Gedächtnis-T-Zellen (T_{CM}-Zellen) umfassen oder wobei die T-Zellen wenigstens 10%, 20%, 30%, 40%, 50% oder 60% naive T-Zellen/Gedächtnis-T-Zellen (T_{N}/_{MEM}-Zellen) umfassen.

10. Zusammensetzung zur Verwendung nach Anspruch 1, wobei nach Behandlung ein erhöhter Spiegel von T-Zellen im ZNS des Patienten nachweisbar ist.

11. Zusammensetzung zur Verwendung nach Anspruch 10, wobei die im CSF nachweisbaren T-Zellen endogene Typ-1-T-Zellen umfassen oder wobei die im CSF nachweisbaren T-Zellen endogene Typ-2-T-Zellen umfassen oder wobei die im CSF nachweisbaren T-Zellen CD3+-T-Zellen umfassen oder wobei die im CSF nachweisbaren T-Zellen reife myeloische CD14+ CD11b+ HLA-DR+-Populationen umfassen oder wobei CD19+-B-Zellen und CD11b+ CD15+-Granulozyten nach Verabreichung der Zusammensetzung im CSF nachweisbar sind oder wobei reaktive Lymphozyten, Monozyten und Makrophagen nach Verabreichung der Zusammensetzung im CSF nachweisbar sind.

12. Zusammensetzung zur Verwendung nach Anspruch 1, wobei der CD19-CAR ein scFv, das an CD19 bindet, eine Spacer-Domäne, eine Transmembrandomäne, wenigstens eine Costimulatordomäne und eine CD3ζ-Signalgebungsdomäne umfasst.

13. Zusammensetzung zur Verwendung nach Anspruch 12, wobei das scFv SEQ ID NO:3 umfasst und/oder wobei die Spacer-Domäne eine von SEQ ID NO:11-22 umfasst und/oder wobei die Transmembrandomäne eine von SEQ ID NO: 22-29 umfasst und/oder wobei die Costimulatordomäne eine von SEQ ID NO: 31-34 umfasst und/oder wobei die CD3ζ-Signalgebungsdomäne SEQ ID NO: 30 umfasst.

14. Zusammensetzung zur Verwendung nach einem der Ansprüche 1-5 oder 7-12, wobei der CD19-CAR die Aminosäuresequenz unter SEQ ID NO: 10 und eine Aminosäuresequenz eines ScFv, das an menschliches CD19 bindet, umfasst.

15. Zusammensetzung zur Verwendung nach einem der Ansprüche 1-14, wobei es sich bei den T-Zellen, die einen CD19-zielgerichteten CAR exprimieren, um T-Zellen handelt, die in einem Kulturmedium mit 60 mg/dl Glucose und 2,8 mÄq/l Kalium kultiviert wurden, oder wobei es sich bei den T-Zellen, die einen CD19-zielgerichteten CAR exprimieren, um T-Zellen handelt, die vor Verabreichung durch In-Kontakt-Bringen mit CD19 aktiviert wurden, oder wobei es sich bei den T-Zellen, die einen CD19-ziel-gerichteten CAR exprimieren, um T-Zellen handelt, die vor Verabreichung durch In-Kontakt-Bringen mit CD19 exprimierenden Zellen aktiviert wurden.

## Revendications

1. Composition comprenant des lymphocytes T exprimant un CAR ciblant CD19 pour utilisation dans un procédé de traitement d'un lymphome du système nerveux central, d'un lymphome systémique avec atteinte concomitante du système nerveux central, d'une leucémie à cellules B du système nerveux central ou d'une leucémie à cellules B systémique avec atteinte concomitante du système nerveux central, la procédé comprenant l'introduction dans le liquide céphalo-rachidien (CSF) d'un patient de ladite composition comprenant une quantité efficace desdits lymphocytes T.

2. Composition pour utilisation selon la revendication 1, dans laquelle les lymphocytes T sont des lymphocytes T autologues ou allogéniques.

3. Composition pour utilisation selon la revendication 1, la composition étant administrée par voie intraventriculaire, ou la composition étant administrée dans le canal central de la moelle épinière.

4. Composition pour utilisation selon la revendication 1, la composition comprenant au moins 1 × 10⁶ cellules, ou une composition comprenant des lymphocytes T étant administrée au moins deux fois.

5. Composition pour utilisation selon la revendication 1, le lymphome étant un lymphome non hodgkinien.

6. Composition pour utilisation selon la revendication 1, dans laquelle le CAR ciblant CD19 comprend une séquence d'acides aminés choisie dans le groupe constitué de SEQ ID NO : 10, 1, 2, 4 et 5.

7. Composition pour utilisation selon la revendication 1, le procédé étant conduit après une greffe myéloablative de cellules souches hématopoïétiques autologues.

8. Composition pour utilisation selon la revendication 1, dans laquelle les lymphocytes T comprennent à la fois des cellules CD4+ et des cellules CD8+, ou dans laquelle les lymphocytes T ont subi une expansion *ex vivo.*

9. Composition pour utilisation selon la revendication 1, dans laquelle les lymphocytes T comprennent au moins 10 %, 20 %, 30 %, 40 %, 50 % ou 60 % de lymphocytes T à mémoire centrale (lymphocytes T_{CM}), ou dans laquelle les lymphocytes T comprennent au moins au moins 10 %, 20 %, 30 %, 40 %, 50 % ou 60 % de lymphocytes T naïfs/lymphocytes T à mémoire (lymphocytes T_{N}/_{MEM}).

10. Composition pour utilisation selon la revendication 1, un taux accru de lymphocytes T étant détectable dans le SNC du patient après traitement.

11. Composition pour utilisation selon la revendication 10, les lymphocytes T détectables dans le LCR comprenant des lymphocytes T endogènes de type 1, ou les lymphocytes T détectables dans le LCR comprenant des lymphocytes T endogènes de type 2, ou les lymphocytes T détectables dans le LCR comprenant des lymphocytes T CD3+, ou les lymphocytes T détectables dans le LCR comprenant des populations myéloïdes matures CD14+ CD11b+ HLA-DR+, ou des lymphocytes B CD19+ et des granulocytes CD11b+ CD15+ étant détectables dans le LCR après administration de la composition, ou des lymphocytes, des monocytes et des macrophages réactifs étant détectables dans le LCR après administration de la composition.

12. Composition pour utilisation selon la revendication 1, dans laquelle le CAR CD19 comprend un scFv qui se lie à CD19, un domaine espaceur, un domaine transmembranaire, au moins un domaine costimulateur et un domaine de signalisation CD3ζ.

13. Composition pour utilisation selon la revendication 12, dans laquelle le scFv comprend SEQ ID NO : 3, et/ou dans laquelle le domaine espaceur comprend l'un quelconque de SEQ ID NO : 11 à 22, et/ou dans laquelle le domaine transmembranaire comprend l'un quelconque de SEQ ID NO : 22-29, et/ou dans laquelle le domaine costimulateur comprend l'une quelconque de SEQ ID NO : 31 à 34, et/ou dans laquelle le domaine de signalisation CD3ζ comprend SEQ ID NO : 30.

14. Composition pour utilisation selon l'une quelconque des revendications 1 à 5 ou 7 à 12, dans laquelle le CAR CD19 comprend la séquence d'acides aminés de SEQ ID NO : 10 et une séquence d'acides aminés d'un ScFv qui se lie à CD19 humain.

15. Composition pour utilisation selon l'une quelconque des revendications 1 à 14, dans laquelle les lymphocytes T exprimant un CAR ciblant CD19 sont des lymphocytes T qui ont été cultivés dans un milieu de culture contenant 60 mg/dl de glucose et 2,8 mEq/l de potassium, ou dans lequel les lymphocytes T exprimant un CAR ciblant CD19 sont des lymphocytes T qui ont été activés par exposition à CD19 avant l'administration, ou dans lequel les lymphocytes T exprimant un CAR ciblé CD19 sont des lymphocytes T qui ont été activés par exposition à des cellules exprimant CD19 avant administration.
